(19) Europäisches Patentamt
European Patent Office
Office européen des brevets

(11) Publication number: 0 014 853
B1

(12) **EUROPEAN PATENT SPECIFICATION**

(45) Date of publication of patent specification: 01.06.83

(51) Int. Cl.³: C 07 H 15/24, A 61 K 31/70

(21) Application number: 80100352.6

(22) Date of filing: 23.01.80

(54) **Anthracycline derivatives, a process for their preparation and pharmaceutical compositions containing them.**

(30) Priority: 03.02.79 JP 11702/79
31.08.79 JP 110255/79

(43) Date of publication of application:
03.09.80 Bulletin 80/18

(45) Publication of the grant of the patent:
01.06.83 Bulletin 83/22

(84) Designated Contracting States:
BE CH DE FR GB IT LU NL SE

(56) References cited:
BE - A - 869 395
FR - A - 2 353 564
FR - A - 2 371 461

(73) Proprietor: Zaidanhojin Biseibutsu Kagaku Kenkyukai
14-23 Kamiosaki 3-chome
Shinagawa-ku Tokyo (JP)

(72) Inventor: Umezawa, Hamao
23 Toyotamakita 4-chome
Nerima-ku Tokyo (JP)
Inventor: Takeuchi, Tomio
5-1-11 Higashigotanda Shinagawa-ku
Tokyo (JP)
Inventor: Naganawa, Hiroshi
3-17 Denenchofuhoncho Ohta-ku
Tokyo (JP)
Inventor: Tatsuta, Kuniaki
26-7 Matsunoki 2-chome Suginami-ku
Tokyo (JP)

(74) Representative: Deufel, Paul, Dr. et al,
Patentanwälte Müller-Boré, Deufel Schön, Hertel
Siebertstrasse 4 Postfach 860720
D-8000 München 86 (DE)

EP 0 014 853 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Anthracycline derivatives, a process for their preparation and pharmaceutical compositions containing them

This invention relates to new derivatives of anthraycline having the general formula I:

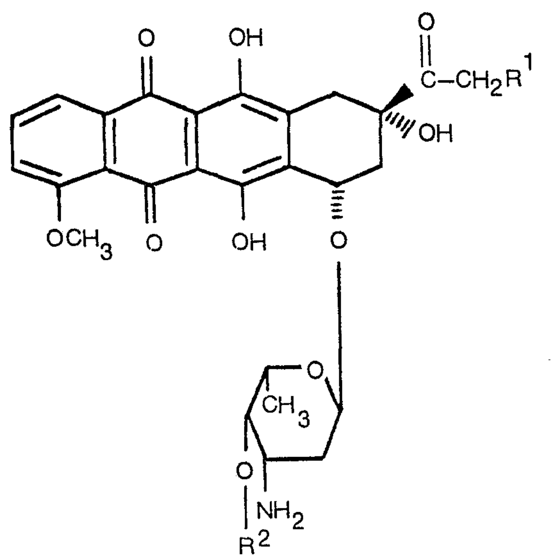

(I)

wherein $R^1$ is a hydrogen atom, a hydroxyl group and $R^2$ is a 1-alkyloxyethyl, a tetrahydrofuran-2-yl, a 6-methoxytetrahydropyran-2-yl, a 6-carbomethoxytetrahydropyran-2-yl or a 6-acetoxy-methyitetra-hydropyran-2-yl group, or acid addition salts thereof, and to a process for producing and recovering the compound having the formula I and a pharmaceutical composition containing thereof.

More particularly, this invention relates to certain new 4'-etherified anthracycline derivatives. The process for producing the compounds according to the present invention which is basically the etherification of the C—4' hydroxyl group of the anthracycline derivative, can be performed by two types of processes according to the difference of the initial material. One comprises etherification with alkyl vinyl ether having a carbon number of 3 to 10, dihydrofuran or dihydropyran derivatives at the C—4' hydroxyl group position of formula II-a:

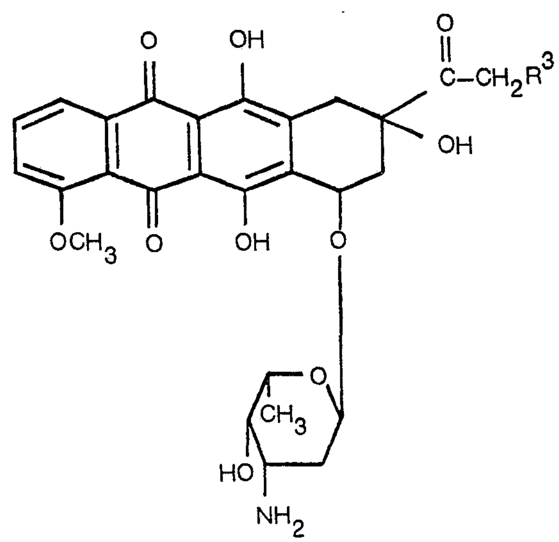

(II-a)

wherein $R^3$ is an alkanoyloxy group such as acetyloxy or isobutyloyloxy, or phenylacetyloxy group, and subsequent deacylation at the C—14 position, and the other comprises the same etherification at the C—4' hydroxyl group position of daunomycin having the general formula IV:

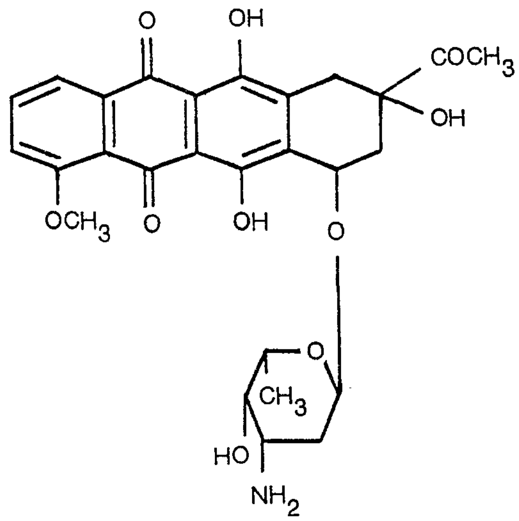

(IV)

Daunomycin (U.K. Patent 1,003,383, U.S. Patent 3,616,242) and adriamycin (U.S. Patent 3,590,028, U.S. Patent 3,803,124), which are obtained from *Streptomyces* cultures, have a broad antitumor spectrum against various experimental tumors, and have been used clinically as potent cancer chemotherapeutic agents. However, it is known that they have severe side effects such as alopecia, leukopenia, cardiotoxicity etc.

The present inventors have extensively studied the chemical modification of daunomycin and adriamycin to prepare more useful derivatives having more active anticancer activity and low toxicity (side effects) than parent compounds, and found that certain derivatives of daunomycin and adriamycin which are substituted at their reactive hydroxyl group at the C—4′ position with alkyloxyethyl, tetrahydrofuranyl or 6-substituted tetrahydropyranyl group have strong anti-cancer activity as well as low toxicity.

Corresponding derivatives of daunomycin and adriamycin wherein the C—4′ hydroxyl group is substituted with a tetrahydropyran-2-yl group are known from BE - A - 869 395.

Among the derivative of the present invention, certain 14-O-acyl-derivatives of adriamycin are also useful as intermediates for obtaining the derivatives of the present invention.

Among new derivatives in the present invention having the general formula I, pharmaceutically useful representative derivatives are as follows.

As examples of the new 4′-O-pyranylated, -furanylated and -alkoxyethylated derivatives of adriamycin the following are mentioned.

4′-O-tetrahydrofuranyl adriamycin
4′-O-tetrahydrofuranyl adriamycin *a* and *b*
4′-O-(1-ethyloxyethyl) adriamycin *a* and *b*
4′-O-(1-butyloxyethyl) adriamycin *a* and *b*
4′-O-(1-isobutyloxyethyl) adriamycin *a* and *b*
4′-O-(1-(6-methylheptyloxy)ethyl) adriamycin *a* and *b*
4′-O-cyclohexyloxyethyl adriamycin *a* and *b*
4′-O-(6-methoxytetrahydropyranyl) adriamycin *a* and *b*
4′-O-(6-carbomethoxytetrahydropyranyl) adriamycin *a* and *b*
4′-O-(6-acetoxytetrahydropyranyl) adriamycin *a* and *b*

As examples of the new derivative of daunomycin, the following are set forth.

4′-O-tetrahydrofuranyl daunomycin *a* and *b*
4′-O-(1-ethyloxyethyl) daunomycin *a* and *b*
4′-O-(1-butyloxyethyl) daunomycin *a* and *b*
4′-O-(1-isobutyloxyethyl) daunomycin *a* and *b*
4′-O-(1-(6-methylheptyloxy)ethyl) daunomycin *a* and *b*
4′-O-cyclohexyloxyethyl daunomycin *a* and *b*
4′-O-(6-methoxytetrahydropyranyl) daunomycin *a* and *b*
4′-O-(6-carbomethoxytetrahydropyranyl) daunomycin *a* and *b*
4′-O-(6-acetoxytetrahydropyranyl) daunomycin *a* and *b*

The process for producing new anthracycline derivatives of formula I in the present invention can be divided into two types as described in the following explanation.

The first type of process for producing the products of the invention comprises introducing an alkyloxyethyl such as 1-methoxyethyl, 1-ethyloxyethyl, 1-butyloxyethyl, 1-isobutyloxyethyl, 1-(6-methylheptyloxy) ethyl or cyclohexyloxyethyl, tetrahydrofuran-2-yl, 6-methoxytetrahydropyran-2-yl, 6-carbomethoxytetrahydropyran-2-yl or a 6-acetoxymethyltetrahydropyran-2-yl group into the hydroxyl

group at the C—4′ position of an anthracycline derivative of the aforementioned (page 2) formula II-a, or an acid addition salt thereof by reaction with dihydrofuran, a dihydropyran derivative or an alkyl vinyl ether which contains said corresponding group to be introduced; and is capable of reacting with a hydroxyl group, in an inert organic solvent and in the presence of acid catalyst to produce a C—4′ etherified derivative of the general formula II:

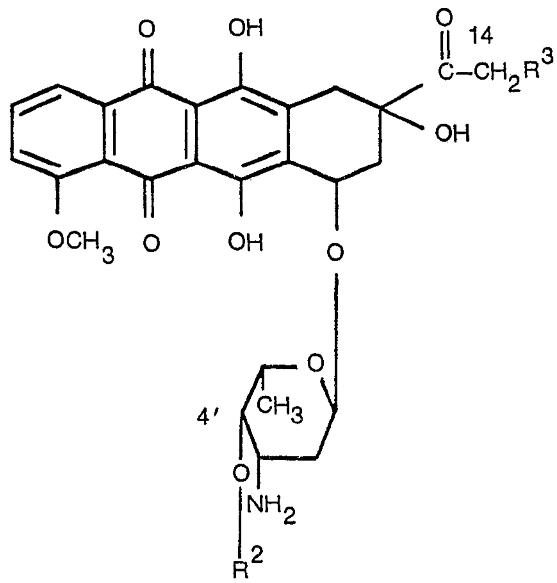

(II)

wherein $R^3$ is an alkanoyloxy group having a carbon number of 2 to 7 or a phenylacetyloxy group, and $R^2$ is a 1-alkyloxyethyl, a tetrahydrofuran-2-yl, a 6-methoxytetrahydrop-2-yl, a 6-carbomethoxytetrahydropyran-2-yl or a 6-acetoxymethyltetrahydropyran-2-yl group or an acid addition salt thereof; and further comprises eliminating the alkanoyl group or the phenylacetyl group at the C—14 position of said derivative of general formula II by hydrolytic deacylation to produce an anthracycline derivative of the general formula III:

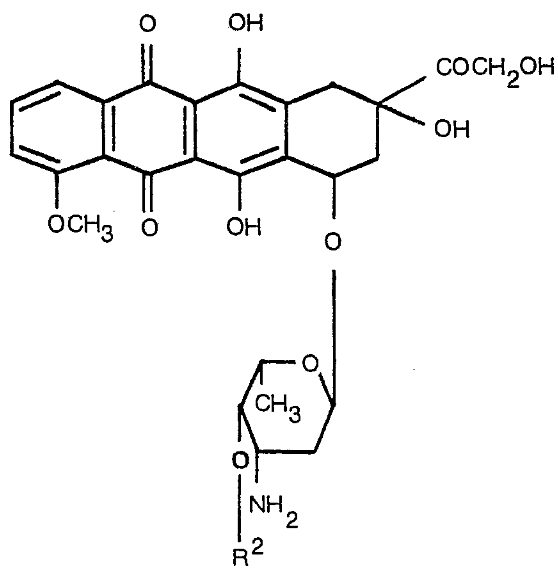

(III)

wherein $R^2$ has the same definition as mentioned above or an acid addition salt thereof.

In the present invention, the compounds (the same compounds as formula II-a) derived from daunomycin by the method as described in Compt. Rend. Acad. de Science t.286, Serie D—443, 1978 and having the general formula VI:

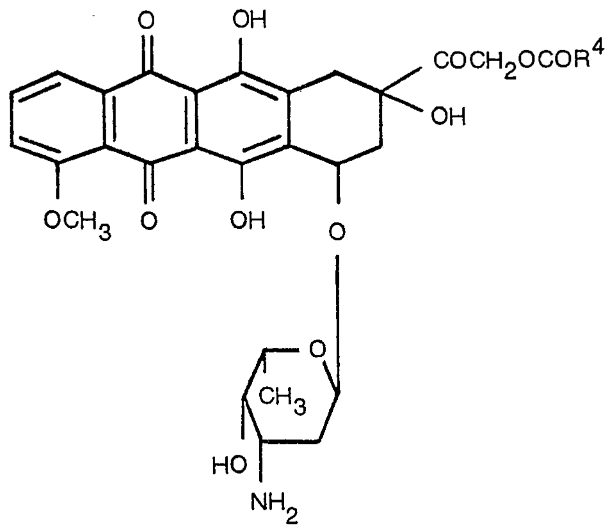

(VI)

wherein $R^4$ is an alkyl group having a carbon number of 1 to 6 or a benzyl group are used as an initial material.

The 14-O-acyl derivatives and 14-O-phenylacetyl derivatives useful as starting materials correspond to the compound obtained by acylation of a hydroxyl group at the C—14 position of adriamycin with alkanoic acid or phenylacetic acid having the formula $R^4COOH$. The intermediates having the general formula VII (the same compounds as formula II):

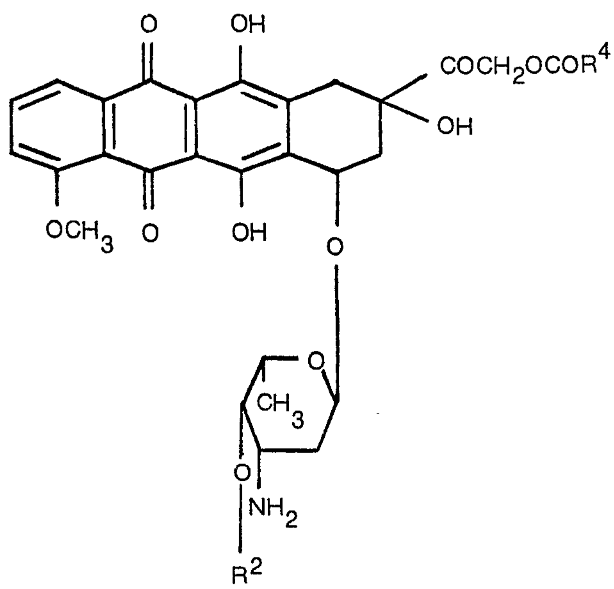

(VII)

wherein $R^4$ is the same group as mentioned above, and $R^2$ is a tetrahydropyran-2-yl, a 6-methoxytetrahydropyran-2-yl, a 6-carbomethoxytetrahydropyran-2-yl, a 6-acetoxytetrahydropyran-2-yl, a tetrahydrofuran-2-yl or a 1-alkyloxyethyl such as 1-methoxyethyl, 1-ethyloxyethyl, 1-butyloxyethyl, 1-isobutyloxyethyl, 1-(6-methylheptyloxy)ethyl or cyclohexyloxyethyl group can be obtained by introducing the corresponding group into the hydroxyl group at the C—4′ position of the compound VI by reaction with dihydrofuran or dihydropyran derivatives or alkyl vinyl ether derivatives having a carbon number of 3 to 10 which contains the corresponding reactive group.

In this case, when the free base of the compound VI or its acid addition salts such as hydrochloride are suspended or dissolved in hereinafter mentioned organic solvents and reacted with dihydropyran derivatives or C3—C10 alkyl vinyl ether derivatives in the presence of an acid catalyst, the reactive hydroxyl group at the C—4′ position of the compound VI can be substituted with tetrahydrofuran-2-yl, tetrahydropyran-2-yl, 6-substituted tetrahydropyran-2-yl, 1-alkyloxyethyl group such as 1-methoxyethyl, 1-ethoxyethyl, 1-butyloxyethyl, 1-isobutyloxyethyl, 1-(6-methylheptyloxy) ethyl or cyclohexyloxyethyl group.

Dihydropyran derivatives such as 2-acetoxyethyl-3,4-dihydro-2H-pyran, 2-methoxy-3,4-dihydro-2H-pyran, 2-carbomethoxy-3,4-dihydro-2H-pyran can be preferably used in the present invention.

Dihydrofuran itself and vinyl ether derivatives such as methyl vinyl ether, ethyl vinyl ether, butyl vinyl ether, isobutyl vinyl ether, 6-methylheptyl vinyl ether or cyclohexyl vinyl ether can be preferably used.

Anhydrous organic solvents such as benzene, toluene dimethylformamide (hereinafter abbreviated as DMF), tetrahydrofuran (hereinafter abbreviated as THF), dimethylsulfoxide (hereinafter abbreviated as DMSO), dioxane and acetonitrile etc. can be preferably used for the reaction either individually or as mixtures thereof. As the acid catalyst, organic sulfonic acids can be used, especially aromatic sulfonic acids such as p-toluenesulfonic acid, benzene sulfonic acid are preferred.

As a preferred example, reaction was carried out with anhydrous DMF as a solvent and p-toluene sulfonic acid as an acid catalyst for 20 min. to 50 hours at room temperature. Alternatively, using such a solvent mixture as that of anhydrous DMSO and anhydrous THF, or anhydrous DMSO and anhydrous dioxane and p-toluenesulfonic acid as the acid catalyst, the reaction can be carried out for 20 min. to 50 hours at room temperature.

To eliminate the acyl group or phenylacyl group at the C—14 position of the compound having the general formula VII (same as formula II), the known method of hydrolysis can be used to obtain the compound having the general formula III. Hydrolysis to remove the $R^4$CO-group at the C—14 position of the compound VII can be achieved by dissolving or suspending the compound VII in water-miscible organic solvent, for example, lower alcohols such as methanol, ethanol etc. and aqueous acetone and reacting at room temperature or under slightly warmer conditions in the presence of alkali, preferably potassium carbonate, at the proper concentration, for example, about 10%(W/V). The completion of hydrolysis can be confirmed by thin-layer chromatography.

Next, the second process aspect of the present invention relates to a process for producing an anthracycline glycoside having the general formula V:

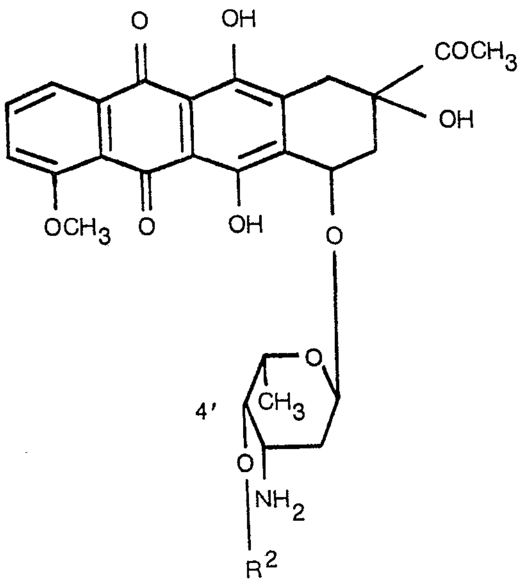

(V)

wherein $R^2$ is a tetrahydrofuran-2-yl, a 6-methoxytetrahydropyran-2-yl, a 6-carboxymethyltetrahydropyran-2-yl, a 6-acetoxytetrahydropyran-2-yl group, or a 1-alkyloxyethyl group such as 1-ethyloxyethyl, 1-butyloxyethyl, 1-isobutyloxyethyl, 1-(6-methylheptyloxy)ethyl or a cyclohexyloxy group or acid addition salts thereof which comprises introducing a tetrahydrofuran-2-yl, a 6-methoxytetrahydropyran-2-yl, a 6-carbomethoxytetrahydropyran-2-yl, a 6-acetoxymethyltetrahydropyran-2-yl group or 1-alkyloxyethyl such a 1-methoxyethyl, 1-ethyloxyethyl, 1-butyloyxethyl, 1-isobutyloxyethyl, 1-(6-methylheptyloxy) ethyl or a cyclohexyloxyethyl group by reaction with dihydropyran derivatives, such as 2-acetoxyethyl-3,4-dihydro-2H-pyran, 2-methoxy-3,4-dihydro-2H-pyran, 2-carbomethoxy-3,4-dihydro-2H-pyran, dihydrofuran or alkyl vinyl ether having a carbon number of 3 to 10, such as methyl vinyl ether, ethyl vinyl ether, butyl vinyl ether, isobutyl vinyl ether, 6-methylheptyl vinyl ether or cyclohexyl vinyl ether capable of reacting with a hydroxyl group, to the hydroxyl group of C—4′ position of daunomycin having the general formula IV:

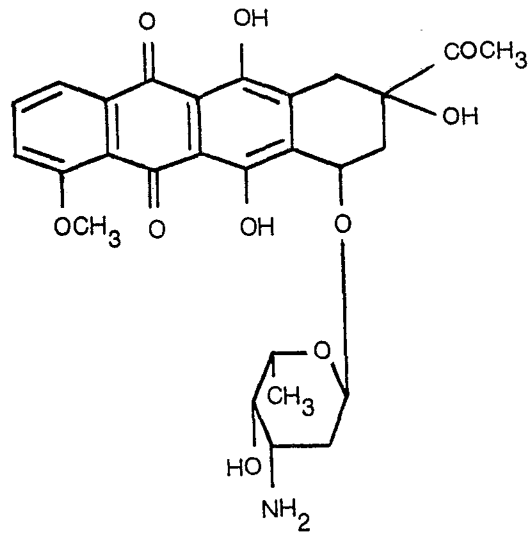

(IV)

or acid addition salts thereof.

In the process, as an initial material the free base of formula IV (daunomycin) or its acid addition salts such as hydrochloride are dissolved or suspended in an organic solvent and reacted with proper dihydropyran derivatives (excluding dihydropyran itself), dihydrofuran or alkyl vinyl ethers having a carbon number of 3 to 10, in the presence of an acid catalyst according to the same method as mentioned previously. Thus, the reactive hydroxyl group at the C—4′ position of the initial material is substituted with a 6-substituted tetrahydropyran-2-yl, a tetrahydrofuran-2-yl, a 1-alkyloxyethyl group, to obtain the above mentioned derivatives of the general formula V. In this case, one or more organic solvents such as benzene, toluene, dimethylformamide (DMF), tetrahydrofuran (THF), dioxane or acetonitrile etc. can be used for the reaction solvent.

Organic sulfonic acids can be used for the acid catalyst, especially aromatic sulfonic acids such as p-toluenesulfonic acid, benzene sulfonic acid are preferred. As a preferred example, reaction was carried out with anhydrous DMF as a solvent, p-toluenesulfonic acid as an acid catalyst for 20 min. to 50 hours at room temperature.

Alternatively, such a mixture as that of anhydrous DMSO and anhydrous THF or anhydrous DMSO and anhydrous dioxane can be preferably used for carrying out the reaction at room temperature for 20 min.—3 hours as mentioned above.

Although the process of the present invention is mentioned separately in the divided form of first and second types for better understanding, they can be unified as follows:

A process for producing an anthracycline derivative of the general formula I′: (combining II and III with V)

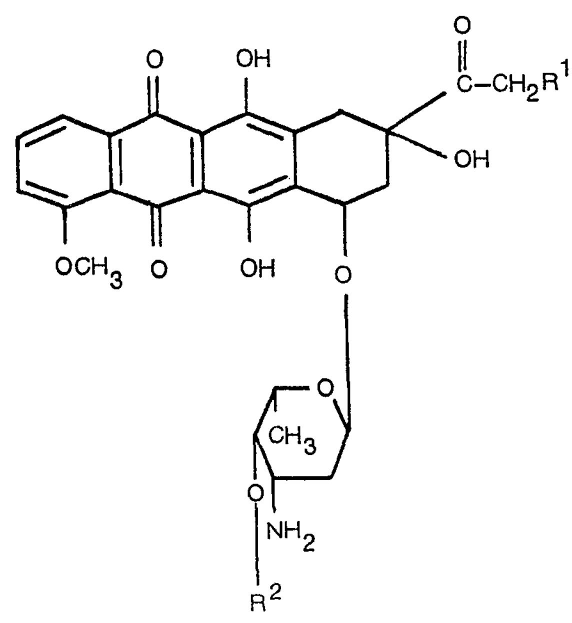

(I′)

wherein $R^1$ is a hydrogen atom or hydroxyl group, $R^2$ is a 1-alkyloxyethyl, a tetrahydrofuran-2-yl, a 6-methoxytetrahydropyran-2-yl, a 6-carbomethoxytetrahydropyran-2-yl or a 6-acetoxymethyltetrahydropyran-2-yl group, or an acid addition salt thereof which comprises introducing a 1-alkyloxyethyl, a tetrahydrofuran-2-yl, a 6-methoxytetrahydropyran-2-yl, a 6-carbomethoxytetrahydropyran-2-yl or a 6-acetoxymethyl tetrahydropyran-2-yl group into the hydroxyl group at the C—4′ position of an anthracycline derivative of the formula VIII: (combining formula II-a with IV)

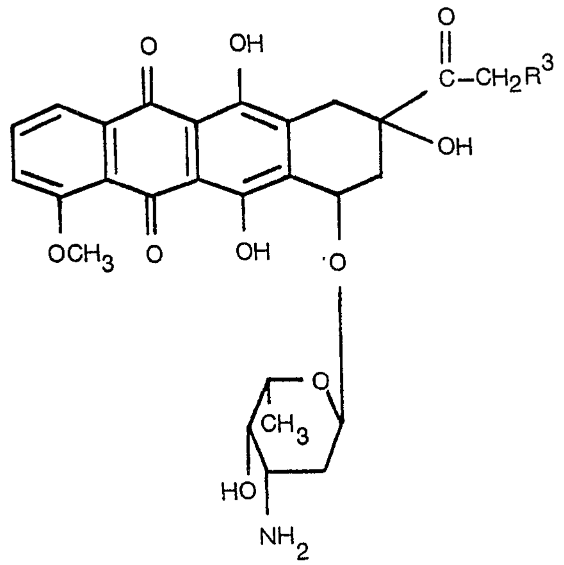

(VIII)

wherein $R^3$ is a hydrogen atom or an alkanoyloxy group having a carbon number of 2 to 7 or a phenylacetyloxy group or an acid addition salt thereof by reaction with dihydrofuran a dihydropyran derivative or a derivative of alkyl vinyl ether which contains said corresponding group to be introduced and is capable of reacting with a hydroxyl group, in an inert organic solvent and in the presence of an acid catalyst to produce the anthracycline derivative of the general formula IX: (combining formula II with V)

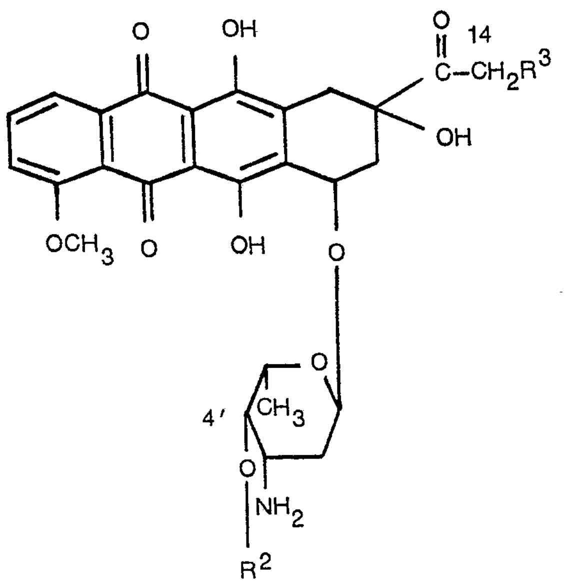

(IX)

wherein $R^3$ is a hydrogen atom or an alkanoyloxy group having a carbon number of 2 to 7 or a phenylacetyloxy group, and $R^2$ is a 1-alkyloxyethyl, a tetrahydrofuran-2-yl, a 6-methoxytetrahydropyran-2-yl, a 6-carbomethoxytetrahydropyran-2-yl or a 6-acetoxymethyltetrahydropyran-2-yl group, or an acid addition salt thereof and, when $R^3$ is other than hydrogen, eliminating the alkanoyl group or the phenylacetyl group at the C—14 position of said derivative of the general formula IX by hydrolytic deacylation to produce an anthracycline derivative of the general formula III:

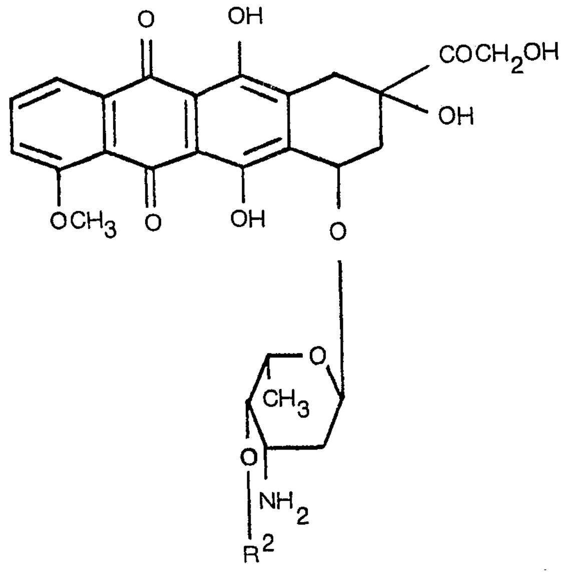

(III)

wherein $R^2$ has the same definition as that mentioned in the formula IX or an acid addition salt thereof.

The compounds obtained above exist as a mixture of diastereomer *a* and *b* in the reaction mixture. If necessary, the diastereomer *a* and *b* can be separated from each other by known methods, and purified from the reaction mixture by conventional methods used for the purification of various anthracycline glycosides. An an example, after filtering the reaction mixture to remove solid materials, the filtrate can be concentrated to dryness and then the resulting crude powder can be purified by column or thin-layer chromatography using alumina, silica gel etc.

The relationship between the structure of the compounds *a* and *b* is considered to be the difference in absolute configuration R and S of the chiral center of the 4′-O-substituted ether groups since both compounds respectively have different chemical shifts of the methine proton of the chiral center.

The compounds in the present invention possess marked antitumor activity against various experimental animal tumors, less toxicity than adriamycin, and can be advantageously used as antitumor agents. Furthermore, the anthracycline derivatives described above wherein $R^1$ is an alkanoyloxy group having a carbon number of 2 to 7 such as an acetyloxy, isobutyloxy or phenylacetyloxy group, are useful as intermediates of the anthracycline derivatives having the hydroxyl group at $R^1$ as above described. Among these derivatives in the present invention, pharmaceutically useful compounds having the general formula I are as follows.

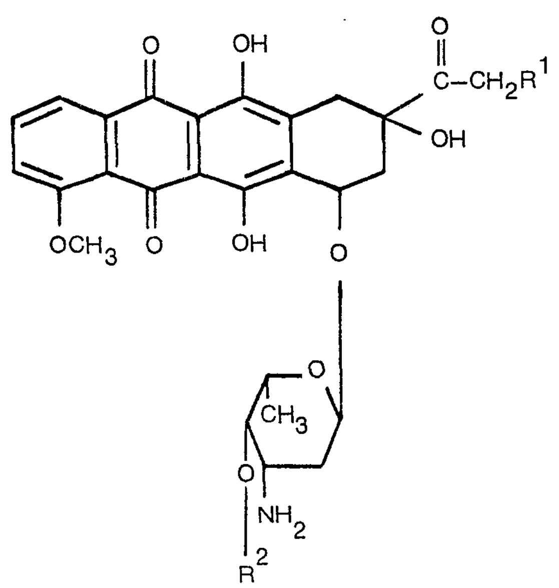

(I)

TABLE 1

| Compounds | No. | $R^1$ | $R^2$ |
|---|---|---|---|
| 4′–O–(tetrahydro-furanyl)–ADM | 2 | –OH | 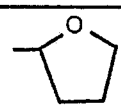 |
| 4′–O–(tetrahydro-furanyl)–ADM (a) | 2-a | ,, | ,, |
| ,, (b) | 2-b | ,, | ,, |
| 4′–O–(6-acetoxy-methyltetrahydro-pyranyl)–ADM | 3 | ,, | 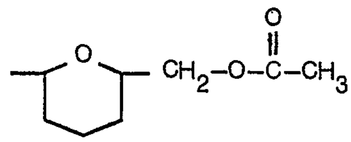 |
| 4′–O–(1-ethoxy-ethyl)–ADM (a) | 4-a | ,, | 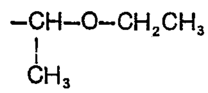 |
| ,, (b) | 4-b | ,, | ,, |
| 4′–O–(1-butyloxy-ethyl)–ADM (a) | 5-a | ,, | 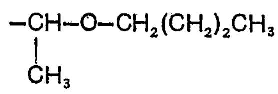 |
| ,, (b) | 5–b | ,, | ,, |
| 4′–O–(1-isobutyloxy-ethyl)–ADM (a) | 6-a | ,, | 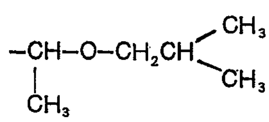 |
| ,, (b) | 6-b | ,, | ,, |
| 4′–O–(1-(6-methyl-heptyloxy) ethyl)-ADM (a) | 7-a | ,, | 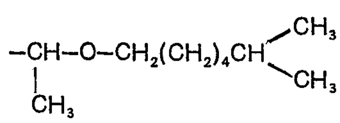 |
| ,, (b) | 7-b | ,, | ,, |
| 4′–O–(1-cyclohexyl-oxyethyl)–ADM (a) | 8-a | ,, | 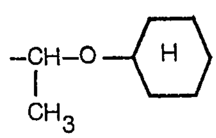 |
| ,, (b) | 8-b | ,, | ,, |
| 4′–O–(tetrahydro-furanyl)–DAM | 9 | –H | 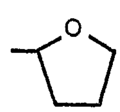 |

TABLE 1 (Continued)

| Compounds | No. | $R^1$ | $R^2$ |
|---|---|---|---|
| 4'–O–(6-methoxy-tetrahydropyranyl)–DAM | 10-a | –H | 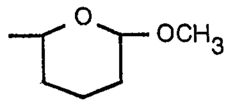 |
| ,, (b) | 10-b | ,, | ,, |
| 4'–O–(6-acetoxy-methyltetrahydro-pyranyl)–DAM | 11 | H | 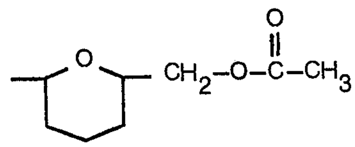 |
| 4'–O–(6-carbo-methoxytetrahydro-pyranyl)–DAM | 12 | ,, | 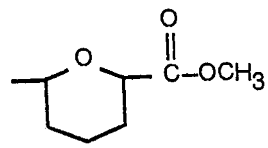 |
| 4'–O–(1-ethoxy-ethyl)–DAM (a) | 13-a | ,, | 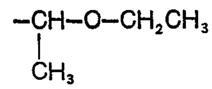 |
| ,, (b) | 13-b | ,, | ,, |
| 4'–O–(1-butyloxy-ethyl)–DAM (a) | 14-a | ,, | 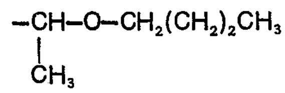 |
| ,, (b) | 14-b | ,, | ,, |
| 4'–O–(1-isobutyloxy-ethyl)–DAM (a) | 15-a | ,, | 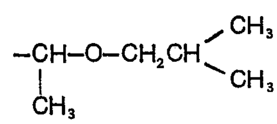 |
| ,, (b) | 15-b | ,, | ,, |

The following describe the pharmacological usefulness of the compounds in the present invention.

(1) Antitumor activity against various experimental animal tumors.

$CDF_1$ mice were inoculated intraperitoneally (i.p.) with $1 \times 10^5$ cells/mouse of L1210 cells. After 24 hours had elapsed since inoculation, the mice were administered the compounds of the present invention intraperitoneally once daily for 10 consecutive days and observed for a 45 day period.

The antitumor activity was shown by the prolongation rate of survival day (T/C, %) to the survival day of control mice injected with physiological saline. The results of the compounds which were numbered in Table 1 are shown in Table 2.

TABLE 2

Antitumor activity (T/C, %) of derivatives in the present invention

| Compounds Nos. | Dose (mg/kg/day) | | | | | |
|---|---|---|---|---|---|---|
| | 5 | 2.5 | 1.25 | 0.625 | 0.31 | 0.15 |
| DAM (for comparison) | Tox. | 138* | 191 | 145 | 132 | 118 |
| ADM (for comparison) | 189* | 351 | 272 | 239 | 147 | 130 |
| 2 | 314 | 360 | 179 | 136 | 111 | 105 |
| 2-a | 212 | 288 | 189 | 135 | 115 | 115 |
| 2-b | 256 | 230 | 154 | 141 | 128 | 122 |
| 3 | 141 | 118 | 118 | 106 | 88 | 100 |
| 4-a | 108 | 277 | 145 | 127 | 101 | 96 |
| 4-b | 211 | 151 | 157 | 127 | 108 | 108 |
| 5-a | 163 | 138 | 125 | 119 | 100 | 100 |
| 5-b | 288 | 138 | 119 | 106 | 100 | 100 |
| 6-a | 257 | 151 | 125 | 112 | 105 | 105 |
| 6-b | 303 | 158 | 118 | 105 | 118 | 99 |
| 7-a | 125 | 125 | 118 | 99 | 92 | 105 |
| 7-b | 118 | 105 | 112 | 112 | 105 | 105 |
| 8-a | 184 | 125 | 118 | 105 | 99 | 105 |
| 8-b | 145 | 125 | 105 | 105 | 105 | 105 |
| 9 | 346 | 160 | 111 | 111 | 105 | 99 |
| 10-a | 232 | 125 | 106 | 100 | 100 | 100 |
| 10-b | 463 | 131 | 113 | 106 | 100 | 94 |
| 12 | 131 | 106 | 100 | 100 | 100 | 100 |
| 13-a | 217 | 157 | 102 | 104 | 96 | 96 |
| 13-b | 247 | 133 | 112 | 108 | 96 | 90 |

TABLE 2 (Continued)

| Compounds Nos. | Dose (mg/kg/day) | | | | | |
|---|---|---|---|---|---|---|
| | 5 | 2.5 | 1.25 | 0.625 | 0.31 | 0.15 |
| 14-a | 144 | 116 | 116 | 110 | 103 | 110 |
| 14-b | 188 | 151 | 110 | 110 | 96 | 103 |
| 15-a | 156 | 119 | 100 | 100 | 100 | 100 |
| 15-b | 188 | 113 | 106 | 100 | 100 | 100 |

Note: DAM = daunomycin, ADM = adriamycin

*: toxic

It is considered from the results of toxic death and body weight loss of mice in Tables 2 and 3 that the derivatives in the present invention are 1/2 to 1/3 lower in toxicity than adriamycin and daunomycin which are the parent compound and the initial material in the present invention.

(2) Inhibitory effect on the growth and nucleic acid biosynthesis in cultured L1210 leukemia cells.

L1210 cells (5 x $10^4$ cells/ml) were inoculated in RPMI 1640 medium (Rosewell Park Memorial Institute 1640) containing 20% calf serum and cultivated at 37°C in the presence of 0.1 and 0.5 μg/ml of the compounds in the present invention in a $CO_2$ incubator. The number of cells were periodically counted and the 50% growth inhibition concentration of control was determined as shown in Table 3.

Furthermore, the 50% inhibition concentration of the compounds in the present invention on nucleic acid biosynthesis was examined as follows:

1 x $10^5$ cells/ml of L1210 cells were suspended in RPMI medium containing 10% calf serum, pre-cultivated at 37°C for 1 to 2 hours in a $CO_2$ incubator and then the compounds in the present invention were added to the medium at various concentrations. After 15 min. of incubation, $^{14}C$-uridine (0.05 μCi/ml) or $^{14}C$-thymidine (0.05 μCi/ml) was added and incubated at 37°C for 60 min. 10% Trichloroacetic acid (TCA) was added to the incubation medium to stop reaction and precipitate the acid-insoluble materials, and then the precipitate was washed three times with 5 to 10% TCA, soluble in formic acid. The radioactivity in the acid-insoluble materials was measured and expressed as 50% inhibition concentration of incorporation. The results are also shown in Table 3.

TABLE 3

Inhibitory effect on the growth and nucleic acid biosynthesis in cultured L1210 cells

| Compounds Nos. | $ID_{50}$ (g/ml) against L1210 cells | | |
|---|---|---|---|
| | growth (after 2 days) | DNA | RNA |
| DAM | 0.036 | 0.3 | 1.7 |
| ADM | 0.018 | 1.25 | 0.49 |
| 2-a | 0.015 | 0.5 | 0.19 |
| 2-b | 0.015 | 0.54 | 0.19 |
| 3 | 0.04 | 0.56 | 0.29 |
| 4-a | 0.015 | 0.36 | 0.13 |
| 4-b | 0.01 | 0.43 | 0.18 |
| 5-a | 0.04 | 1.9 | 0.52 |
| 5-b | 0.05 | 1.5 | 0.37 |
| 6-a | 0.025 | 0.85 | 0.32 |
| 6-b | 0.03 | 0.5 | 0.15 |
| 7-a | 0.38 | >10.0 | 2.5 |
| 7-b | 0.52 | >10.0 | 2.8 |
| 8-a | 0.04 | 1.3 | 0.43 |
| 8-b | 0.06 | 1.3 | 0.32 |
| 9 | 0.017 | 0.41 | 0.17 |
| 10-a | 0.04 | 0.62 | 0.3 |
| 10-b | 0.025 | 0.8 | 0.33 |
| 12 | 0.095 | 0.93 | 0.44 |
| 13-a | 0.025 | 0.34 | 0.13 |
| 13-b | 0.03 | 0.65 | 0.29 |
| 14-a | 0.08 | 0.93 | 0.37 |
| 14-b | 0.075 | 1.4 | 0.6 |
| 15-a | 0.08 | 1.4 | 0.5 |
| 15-b | 0.06 | 0.8 | 0.28 |

Note: DAM = daunomycin, ADM = adriamycin.

(3) Inhibitory effect on the growth of various microorganisms.

The compounds of formula 1 and their nontoxic acid addition salts have a remarkable bacteriostatic activity which is similar to that of adriamycin against several microorganisms, such as *Staph. aureus, Bacillus subtilis, B. cereus, B. megaterium, Sarcina lutea, Micrococcus flavus, Corynebacterium bovis, Pseudomonas aeruginosa, Escherichia coli, Mycobacterium smegmatis* and *Candida albicans.*

Therapeutic Use

As mentioned above, the compounds of formula I and their nontoxic acid addition salts are novel antibiotics, useful in both human and veterinary medicine, and also possess marked inhibitory action against malignant mammalian tumors, including both solid and ascitic types.

According to another aspect of the invention, a pharmaceutical composition is provided which comprises a therapeutically effective antimicrobial or tumor-inhibiting amount of the compounds of formula I or a mixture thereof, or a nontoxic acid addition salt thereof, in combination with a pharmaceutical carrier or diluent. Such compositions may be made up in any pharmaceutical form appropriate for parenteral administration.

Preparations for parenteral administration include sterile aqueous or non-aqueous solutions, suspensions or emulsions. They may also be manufactured in the form of sterile solid compositions which can be dissolved in sterile water, physiological saline or some other sterile injectable medium immediately before use.

It will be appreciated that the actual preferred dosage amounts used will vary according to the particular compound being used, the particular composition formulated, the mode of application and the particular situs, host and disease being treated. In general the compounds are injected intraperitoneally, intravenously, subcutaneously or locally into non-human mammals and intravenously or locally into humans. Many factors that modify the action of the drug will be taken into account by those skilled in the art, for example, age, body weight, sex, diet, time of administration, route of administration, rate of excretion, condition of the patient, drug combinations, reaction sensitivities and severity of the disease. Administration can be carried out continuously or periodically within the maximum tolerated dose. Optimal application rates for a given set of conditions can be ascertained by those skilled in the art using conventional dosage determination tests in view of the above guidelines.

Prior to the description of the Examples, as an example for preparing 14-O-acylated adriamycin intermediate which is to be used as an initial material for the preparation of 14-O-acyl-4′-O-etherified adriamycin, the experimental embodiment of the preparation of 14-O-acetyl-adriamycin will be mentioned as follows. Other 14-O-acylated adriamycin to be used as an initial material of the present invention can be prepared according to this experiment.

Experiment 1

(1) 14 Bromodaunomycin-dimethylketal hydrochloride

230 mg (0.41 mmoles) of daunomycin hydrochloride was dissolved in 10 ml of absolute methanol, adding 20 ml of dioxane. The mixture was allowed to stand for 2 to 3 hours at 22 to 23°C, followed by adding drop-wisely 0.88 ml (0.55 mmoles) of 10% chloroform containing bromine aqueous solution while stirring. The reaction mixture was concentrated to about 5 ml under reduced pressure and thereafter adding 15 ml of dry ether to obtain an orange-red precipitate by filtration. The precipitate was washed three times with 3 ml of ether and dried. 277 mg of hydrochloride salt of 14-O-bromodaunomycin-dimethyl ketal having a melting point at 175 to 178°C was obtained with a yield of 98%.

(2) p-toluenesulfonate or hydrochloride salt of 14-O-acetyl adriamycin

207 mg (0.3 mmoles) of 14-bromodaunomycin dimethylketal hydrochloride salt was suspended in 100 ml of dry acetone, thereafter adding 0.4 g of dry sodium acetate and refluxed for 1 hour with agitation. The reaction mixture was filtered to remove impurities, and the filtrate was concentrated under reduced pressure. The residue was distributed in a mixture containing 30 ml of chloroform and 20 ml of 0.05N hydrochloric acid. The acidic aqueous layer thus obtained was neutralized with sodium hydrogen carbonate and then re-extracted with chloroform. The reddish chloroform layer was dried over anhydrous sodium sulfate and concentrated to about 5 ml under reduced pressure, and an orange precipitate of salt form was formed by adding 40 mg of p-toluenesulfonic acid or an equivalent amount of hydrochloric acid, according to the salification method of the corresponding acid addition salt. The resulting precipitate was filtered and washed with ether, and 160 mg of p-toluenesulfonate salt of 14-O-acetyl adriamycin having a melting point at 165 to 168°C was obtained. In the case of hydrochloride salt, about the same amount of compound can be obtained.

The following examples are provided for illustrative purposes only and are not intended to limit the scope of the invention. In this example, the ratio of solvent in a mixture is indicated in the ratio of volume to volume and the % of liquid to liquid is indicated as V/V and that of solid to liquid is indicated as W/V unless otherwise mentioned.

For the convenience of reading and locating the attributes of NMR, the configuration of adriamycin with indications showing the position of the carbon atoms is indicated below.

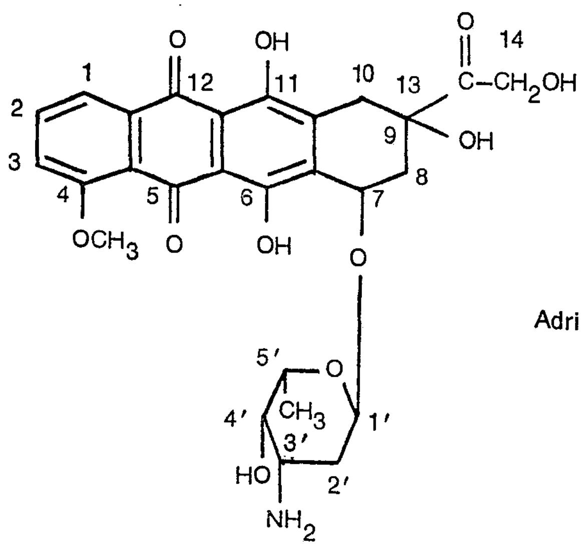

Adriamycin

Example 1

Process for producing 4′-O-tetrahydrofuranyl adriamycin

(a) 190 mg (0.25 mmoles) of 14-O-acetyl adriamycin p-toluenesulfonate was dissolved in 10 ml of dried dimethylformamide, adding 0.4 ml of dihydrofuran and p-toluenesulfonic acid as a catalyst, and allowed to stand for 3.5 hours at room temperature (4′-O-tetrahydrofuranylation). The formation of the reaction product having an Rf of 0.24 to 0.27 and the disappearance of the initial material were observed by silica gel thin-layer chromatography using a solvent system of chloroform-methanol (9:1). The reaction mixture was poured into 100 ml of water, neutralized with sodium hydrogen carbonate and extracted with 60 ml of chloroform. The chloroform layer was dried over anhydrous sodium sulfate, concentrated to dryness under reduced pressure and developed by preparative thin-layer chromatography using two silica gel plates (20×20 cm, 2 mm thickness) and a solvent system of chloroform-methanol (9:1). The band of Rf 0.24 to 0.27 was scratched out from the silica gel thin-layer, extracted with a chloroform-methanol mixture (1:1), and concentrated to dryness. 66.8 mg of 14-O-acetyl-4′-O-tetrahydrofuranyl adriamycin was obtained as a red solid.

Yield 40%

PMR ($CDCl_3$, ppm): 1.23—1.34(6′—H), 1.70—2.12(furan), 2.20, 2.22(acetyl), 4.06(4-OMe), 5.20(14—H), 5.36(furan, anomeric), 5.51(7—H), 7.27—8.06(1—H — 3—H)

(b) 41.0 mg of 14-O-acetyl-4′-O-tetrahydrofuranyl adriamycin was dissolved in 10 ml of methanol and 3 ml of water while stirring, adding 10% potassium carbonate solution to change the solution to blue-violet color (pH 10—11) and allowed to stand for 30 min. (hydrolysis). Descending of the Rf value was obserbed by silica gel thin-layer chromatography using a solvent system of chloroform-methanol (1:1). The reaction mixture was neutralized by adding a small piece of dry-ice and extracted with chloroform. The chloroform layer was washed with water, dried and concentrated to dryness. The resulting residue was purified by preparative thin-layer chromatography according to the method of (a). 16.3 mg of 4′-O-tetrahydrofuranyl adriamycin was obtained as red powder.

Yield 43%, Melting point (°C): 189—194 (decomposition)

PMR ($CDCl_3$, ppm): 1.25—1.27(6′—H), 1.67—2.30 (furan), 4.07—4.08(4—H), 5.17 and 5.38(furan, anomeric), 5.30(1′—H), 5.51(7—H), 7.30—8.07(1—H — 3—H)

Example 2

Process for producing 4′-O-tetrahydrofuranyl adriamycin *a* and *b*

40 mg of 4′-O-tetrahydrofuranyl adriamycin obtained in Example 1 was preparatively rechromatographed using silica gel (Merck Co.) and a solvent system of chloroform-methanol (15:1). An amount less than 1 mg per plate was applied on a silica gel plate (0.25 mm thickness, 20×20 cm), and developed three times to achieve good separation. The band corresponding to Rf 0.20 was scratched out from the silica gel plate, eluted with chloroform-methanol (1:1) and concentrated to dryness. The resulting compound *a* weighed 11.0 mg and contains the following properties.

Melting point (°C): 189—191, $[\alpha]_D$+175° ($CHCl_3$, C=0.2)

PMR ($CDCl_3$, ppm): 1.25(6'—H), 1.67—2.37(furan), 4.07(4—H), 5.17(furan, anomeric), 5.30(1'—H), 5.51(7—H), 7.30—8.06(1—H — 3—H)

The fractions of Rf 0.22 weighed 12.1 mg of compound *b*, and its properties are as follows:

Melting point (°C): 190—192 $[\alpha]_D$+150° ($CHCl_3$, C=0.2)

PMR ($CDCl_3$, ppm): 1.27(6'—H), 1.67—2.30(furan), 4.08(4—H), 5.30(1'—H), 5.38(furan, anomeric), 5.51(7—H), 7.30—8.07(1—H — 3—H)

Example 3

Process for producing 4'-O-(6-acetoxymethyltetrahydropyranyl) adriamycin

(a) 150 mg (0.18 mmoles) of p-toluenesulfonate of 14-O-phenylacetyl adriamycin was dissolved in 2.0 ml of dry dimethylformamide, adding 0.2 ml of 2-acetoxymethyl-3,4-dihydro-2H-pyran and 15 mg (0.09 mmoles) of p-toluenesulfonic acid and allowed to stand for 24 hours at room temperature (4'-O-(6-acetoxymethyltetrahydropyranylation)]. The spots of the initial material and the product having a Rf 0 51 was detected in the reaction mixture by silica gel thin-layer chromatography using a solvent system of chloroform-methanol (9:1). The reaction mixture was poured into 20 ml. of water, neutralized with sodium hydrogen carbonate and extracted twice with 30 ml of chloroform. The chloroform layer was dried over anhydrous sodium sulfate and chromatographed on 10 g of silica gel column (Merck Co. Kiesel gel #100), and the column was washed with 100 ml of chloroform. The product was successively eluted with a chloroform-methanol mixture (10:1) and detected by thin-layer chromatography. The fraction of Rf 0.51 was collected, concentrated to dryness, and 100.2 mg of 14-O-phenylacetyl-4'-O-(6-acetoxymethyltetrahydropyranyl) adriamycin was obtained as red solid.

Yield 68%

PMR ($CDCl_3$, ppm): 1.24—1.34(6'—H), 2.08(acetyl), 3.83, 7.40(phenylacetyl), 4.08(4'—H), 4.95(pyran, anomeric), 5.25(14—H), 5.54(7—H), 7.30—8.08(1—H — 3—H)

(b) Then, the substance obtained above was dissolved in 20 ml of acetone with agitation, adding 10 ml of water and 0.5 ml of 10% potassium carbonate, and hydrolyzed for 30 min. The product of Rf 0.41 was observed on a thin-layer chromatogram. A piece of dry-ice was added to the reaction mixture for neutralizing and distilled to remove the acetone under reduced pressure. The resulting aqueous layer was extracted three times with 10 ml of chloroform respectively. The chloroform layers were combined, washed with water, dried over anhydrous sodium sulfate, and concentrated to dryness to obtain a red solid. The solid was further purified by preparative thin-layer chromatography using silica gel (Merck Co. 2 mm thickness and 20×20 cm, one plate) and a solvent system of chloroform-methanol (9:1), and re-extracted from the fraction of Rf 0.41. 26.9 mg of 4'-O-(6-acetoxymethyltetrahydropyranyl) adriamycin was obtained as red powder.

Yield 31%, Melting point (°C): 174—178

PMR ($CDCl_3$, ppm): 1.19—1.40(6'—H), 2.06, 2.10(OAc), 4.11(4—OMe), 4.78(14—H), 5.55(7—H), 7.35—8.10(1—H — 3—H)

Example 4

Process for producing 4'-O-(1-ethyloxyethyl) adriamycin *a* and *b*

(a) 200 mg of p-toluenesulfonate of 14-O-phenylacetyl adriamycin was dissolved in 4.0 ml of dry dimethyl formamide, adding 0.2 ml of ethyl vinyl ether and p-toluenesulfonic acid as a catalyst, and allowed to stand for 1.5 hours at room temperature (4'-O-ethyloxyethylation). By silica gel thin-layer chromatography (solvent system; chloroform-methanol = 9:1) of the reaction mixture, it was confirmed that the initial material disappeared and new products of Rf 0.36 and 0.39 were formed. The reaction mixture was poured into 20 ml of water, adding sodium hydrogen carbonate to adjust the Ph at 8, and extracted with 60 ml of chloroform. The chloroform layer was dried over anhydrous sodium sulfate and concentrated under reduced pressure to obtain a red material. After purifying the material by preparative thin-layer chromatography using silica gel (2 mm thickness, 20×20 cm, 2 plates) and a solvent system of chloroform-methanol (9:1), 134 mg of 14-O-phenylacetyl-4'-O-(1-ethyloxyethyl) adriamycin was obtained from the band of Rf 0.36—0.39 as red powder.

Yield 76%

PMR ($CDCl_3$, ppm): 1.21, 3.62(1'-O-ethyl), 1.40(2'—H), 3.81, 7.38(phenyl-acetyl), 4.02(4-O-methyl), 5.64, 5.94(1'—H), 5.24(14—H), 5.50(7—H), 7.25—8.02(1—H — 3—H)

(b) Then, the compound obtained above was dissolved in 40 ml of acetone, adding 20 ml of water and 150 mg of anhydrous potassium carbonate and agitated well (hydrolysis). It was confirmed by silica gel thin-layer chromatography (solvent system, chloroform-methanol = 9:1) that the substances of Rf 0.36 and 0.39 gradually disappeared and new products of Rf 0.28 and 0.21 were formed. After 20 min. of reaction, the reaction mixture was neutralized with a piece of dry-ice and distilled to remove the acetone. The resulting aqueous layer was extracted twice with 30 ml of chloroform respectively, and the chloroform layer was washed with water, dried over anhydrous sodium sulfate and concentrated to obtain a red residue. According to the same method as mentioned above, the residue was purified by preparative silica gel thin-layer chromatography using a chloroform-methanol mixture (once with 15:1 and twice 9:1 to achieve good separation). The band of Rf 0.21 was scratched out from silica gel thin-layer and extracted with a chloroform-methanol mixture (1:1), and the extract was concentrated to obtain 13.0 mg of red powder of 4'-O-(1-ethyloxyethyl) adriamycin *a*.

Yield 13%, Melting point (°C): 205—215

PMR ($CDCl_3$, ppm): 1.15, 3.58(1—OEt), 1.40(2'—H), 3.58(2'—H), 4.06(4—OMe), 4.65(1'—H), 4.76(14—H), 5.25(1'—H), 5.51(7—H), 7.25—8.07(1—H — 3—H)

11.2 mg of 4'-O-(1-ethyloxyethyl) adriamycin *b* was obtained from the band of Rf 0.28.

Yield 11%, Melting point (°C): 190—200

PMR ($CDCl_3$, ppm): 1.22, 3.63(1'—OEt), 4.08(4—H), 4.76(14—H), 4.93(1'—H), 5.27(1'—H), 5.50(7—H), 7.22—8.88(1—H — 3—H)

## Example 5

### Process for producing 4'-O-(1-butyloxyethyl) adriamycin *a* and *b*

(a) 145.6 mg of p-toluenesulfonate of 14-acetyl adriamycin was dissolved in a mixture of 2 ml of absolute DMSO and 4 ml of absolute dioxane, adding 1.6 ml of n-butyl vinyl ether and further adding 0.396 ml of 0.1N p-toluenesulfonic acid-dioxane solution under ice-cold conditions. After agitation for 35 min. at room temperature, the reaction mixture was poured into 50 ml of ethylacetate and washed twice with 30 ml of 1% sodium hydrogen carbonate solution respectively and three times with 30 ml of 5% sodium chloride aqueous solution respectively. The aqueous layer was extracted with 50 ml of ethylacetate, and the solvent extracts were combined and dried over anhydrous sodium sulfate, and concentrated under reduced pressure to obtain an oily substance.

The oily substance was chromatographed on the silica gel column (Merck Art. 77 34, 4 g, solvent: (1) $CHCl_3$ 60 ml, (2) $CHCl_3$: methanol=10:1 30 ml) to obtain 141 mg from the eluate of the chloroform-methanol mixture. This product was further purified by preparative thin-layer chromatography (Merck Art. 5744, solvent system: $CHCl_3$: methanol=10:1) to obtain 4'-O-(1-butyloxyethyl) adriamycin *a* and *b*.

Compound *a*: 33.5 mg

Compound *b*: 40.5 mg

(b) Deacetylation

40.5 mg of 4'-O-(1-butyloxyethyl) adriamycin *b* was dissolved in 4 ml of methanol and 1 ml of water, and adding 0.23 ml of 1N potassium carbonate aqueous solution to change the solution to blue-violet color. After 8 min. of reaction, the reaction mixture was neutralized with a piece of dry-ice, adding 60 ml of water, extracted with 30 ml and 20 ml × 3 of chloroform, dried over anhydrous sodium sulfate and concentrated under reduced pressure. By preparative thin-layer chromatography (Merck Art. 5744, solvent system: $CHCl_3$: methanol=10:1), 14.2 mg of 4'-O-(1-butyloxyethyl) adriamycin b and 10.7 mg of compound *a* were obtained.

| Compound *a* | |
|---|---|
| Melting point (°C): | 168—175 |
| Specific rotation (C=0.1 in chloroform) $[\alpha]_D^{25}$: | +157° |

| | |
|---|---|
| UC and visible absorption spectra in methanol $\lambda max_{nm}[E^{1\%}_{1cm}]$: | 234(585, 251(405) 288(155), 480(187) 495(190), 530(116) 575(19) |
| PMR ($CDCl_3$, ppm): | 0.92 (t, 3H) —$CH_2CH_3$ |
| | 1.31 (d, 3H) C5′—$CH_3$ |
| | 1.38 (d, 3H) C4′—$OCHCH_3$ |
| | 2.91 (d, 1H) } C10—$CH_2$ 3.21 (d, 1H) |
| | 4.03 (s, 3H) C4—$OCH_3$ |
| | 4.74 (s, 2H) C14—$CH_2$ |
| | 4.89 (q, 1H) C4′—$OCHCH_3$ |
| | 5.22 (bs, 1H) C7—H |
| | 5.48 (bs, 1H), C1′—H |
| | 7.25—7.98 (m, 3H) |
| | aromatic proton |

<u>Compound *b*</u>

| | |
|---|---|
| Melting point (°C): | 155—160 |
| Specific rotation (C=0.1 in chloroform) $[\alpha]^{25}_D$: | +188° |
| UV and visible absorption spectra in methanol $\lambda max_{nm}(E^{1\%}_{1cm})$: | 234(533), 251(371) 288(140), 480(168) 495(172), 530(106) 575(19) |
| PMR ($CDCl_3$, ppm): | 0.91 (t, 3H) —$CH_2CH_3$ |
| | 1.27 (d, 3H) C5′—$CH_3$ |
| | 1.40 (d, 3H) C4′—$OCHCH_3$ |
| | 2.95 (d, 1H) } C10—$CH_2$ 3.26 (d, 1H) |
| | 4.06 (s, 3H) C4—$OCH_3$ |
| | 4.61 (q, 1H) C4′—$OCHCH_3$ |
| | 4.74 (s, 2H) C14—$CH_2$ |
| | 5.27 (bs, 1H) C7—H |
| | 5.50 (bs, 1H) C1′—H |
| | 7.31—8.02 (m, 3H) |
| | aromatic proton |

Example 6

Process for producing 4'-O-(1-isobutyloxy) adriamycin *a* and *b*

(a) 150 mg of p-toluenesulfonate of 14-O-acetyl adriamycin was dissolved in 3 ml of anhydrous dioxane and 1.5 ml of anhydrous dimethylsulfoxide, adding 0.7 ml of isobutyl vinyl ether and 0.3 ml of 0.1N p-toluenesulfonic acid-dioxane solution, and agitated for 40 min. at room temperature (23°C). The reaction mixture was diluted with 50 ml of ethylacetate, washed subsequently with 1% sodium hydrogen carbonate aqueous solution (30 ml x 2) and water (30 ml x 2), dried over anhydrous sodium sulfate and concentrated under reduced pressure. The resulting oily substance was chromatographed on silica gel column (Merck Art. 7734, 4g; $CHCl_3 \rightarrow CHCl_3$: methanol=10:1), and the fractions containing the main product were collected and concentrated to dryness to obtain 125.5 mg of orange powder. The powder was subjected to preparative thin-layer chromatography (Merck Art. 5744, 12 plates, $CHCl_3$: methanol=10:1) to separate and purify, and 14-O-acetyl-4'-O-(1-isobutyloxyethyl) adriamycin *a* and *b* were obtained as follows:

Compound *a*: 40.4 mg

Compound *B*: 51.7 mg

(b) Deacetylation at C—14 position

40.4 mg of 14-O-acetyl-4'-O-(1-isobutyloxyethyl) adriamycin *a* obtained in (a) was dissolved in 4 ml of methanol, adding 2 ml of water and 0.36 ml of 1N potassium carbonate aqueous solution and agitated for 20 min. at room temperature. The reaction mixture was neutralized with dry-ice, extracted with 60 ml of chloroform and then washed with 50 ml of 5% sodium chloride aqueous solution. The aqueous layer was further extracted twice with 20 ml of chloroform respectively, and the combined chloroform extracts were dried over anhydrous sodium sulfate and concentrated to dryness. 35.8 mg of the resulting residue was chromatographed by preparative thin-layer chromatography (Merck Art. 5744, 4 plates, $CHCl_3$: methanol=10:1) to obtain 12.5 mg of 4'-O-(1-isobutyloxyethyl) adriamycin *a*.

| | |
|---|---|
| Melting point (°C): | 180—185 (decomposition) |
| Specific rotation (C=0.1 in chloroform) $[\alpha]_D^{25}$: | +157° |
| PMR ($CDCl_3$, ppm): | 0.93 (d, 6H) $CH(CH_3)_2$ |
| | 1.32 (d, 3H) C5'—$CH_3$ |
| | 1.38 (d, 3H) C4'—$OCHCH_3$ |
| | 2.86 (d, 1H), 3.16 (d, 1H) } C10—$CH_2$ |
| | 3.66 (bs, 1H) C4'—H |
| | 4.02 (s, 3H) C4—$OCH_3$ |
| | 4.74 (s, 2H) C14—$CH_2$ |
| | 4.88 (q, 1H) C4'—$OCHCH_3$ |
| | 5.18 (bs, 1H) C7—H |
| | 5.48 (bs, 1H) C1'—H |
| | 7.25—7.92 (m—3H) aromatic proton |

51.7 mg of 14-O-acetyl-4'-O-(1-butyloxyethyl) adriamycin *b* obtained in (a) was dissolved in 5 ml of methanol, adding 1.5 ml of water and 0.39 ml of 1N potassium carbonate aqueous solution and agitated for 15 min. at room temperature. According to the method for compound *a*, 18.1 mg of 4'-O-(1-butyloxyethyl) adriamycin *b* was obtained.

| | |
|---|---|
| Melting point (°C): | 175—180 (decomposition) |
| Specific rotation (C=0.1 in chloroform) $[\alpha]_D^{25}$: | +191° |
| PMR ($CDCl_3$, ppm): | 0.90 (d, 6H) $CH(CH_3)_2$ |
| | 1.27 (d, 3H) C5′—$CH_3$ |
| | 1.40 (d, 3H) C4′—$OCHCH_3$ |
| | 2.84 (d, 1H) } C10—$CH_2$ |
| | 3.16 (d, 1H) } |
| | 3.26 (t, 2H) $OCH_2CH$ |
| | 3.58 (bs, 1H) C4′—H |
| | 4.01 (s, 3H) C4—$OCH_3$ |
| | 4.60 (q, 1H) 4′—$OCHCH_3$ |
| | 4.74 (s, 2H) C14—$CH_2$ |
| | 5.18 (bs, 1H) C7—H |
| | 5.48 (bs, 1H) C1′—H |
| | 7.25—7.90 (m, 3H) |
| | aromatic proton |

Example 7

Process for producing 4′-O-(1-(6-methylheptyloxy)ethyl) adriamycin *a* and *b*

(a) 150 mg of 14-O-acetyl adriamycin p-toluenesulfonate was dissolved in 2 ml of absolute DMSO and 4 ml of absolute dioxane, adding 1.6 ml of 6-methyl-heptyl vinyl ether and 0.396 ml of 0.1N p-toluenesulfonic acid-dioxane solution under ice-cold conditions, and agitated for 45 min. at room temperature. According to the same method as that of (a) in Example 5, 14-O-acetyl-4′-O-(6-methyl-heptyloxyethyl) adriamycin *a* and *b* were obtained.

Compound *a:* 32.7 mg

Compound *b:* 57.4 mg

(b) Deacetylation

44.2 mg of 14-O-acetyl-4′-O-(1-(6-methylheptyloxy)ethyl) adriamycin *b* was dissolved in 5.8 ml of methanol and 1.3 ml of water, adding 0.39 ml of 1N potassium carbonate solution to obtain a blue-violet color, and allowed to stand for 6 min. at room temperature. According to the same method as that of (b) in Example 5, 20.1 mg of 4′-O-(6-methylheptyloxy)ethyl adriamycin *b* was obtained. 13 mg of compound *a* was obtained in the same manner as mentioned above.

<u>Compound *a*</u>

| | |
|---|---|
| Melting point (°C): | 143—147 |
| Specific rotation (C=0.1 in chloroform) $[\alpha]_D^{25}$: | +178° |

| | |
|---|---|
| PMR ($CDCl_3$, ppm): | 1.32 (d, 3H) C5′—$CH_3$ |
| | 1.38 (d, 3H) C4′—OCHC$H_3$ |
| | 2.96 (d, 1H) } C10—$CH_2$ |
| | 3.26 (d, 1H) } |
| | 4.06 (s, 3H) C4—$OCH_3$ |
| | 4.75 (s, 2H) C14—2H |
| | 4.90 (q, 1H) C4′—OC*H*$CH_3$ |
| | 5.28 (bs, 1H) C7—H |
| | 5.51 (bs, 1H) C1′—H |
| | 7.31—8.03 (m, 3H) |
| | aromatic proton |

Compound *b*

| | |
|---|---|
| Melting point (°C): | 145—150 |
| Specific rotation (C=0.1 in chloroform) $[\delta]_D^{25}$: | +183° |
| PMR ($CDCl_3$, ppm): | 1.27 (d, 3H) C5′—$CH_3$ |
| | 1.40 (d, 3H) C4′—OCHC$H_3$ |
| | 2.93 (d, 1H) } C10—$CH_2$ |
| | 3.25 (d, 1H) } |
| | 4.06 (s, 3H) C4—$OCH_3$ |
| | 4.61 (q, 1H) C4′—OC*H*$CH_3$ |
| | 4.75 (s, 2H) C14—$CH_2$ |
| | 5.25 (bs, 1H) C7—H |
| | 5.52 (bs, 1H) C1′—H |
| | 7.30—8.00 (m, 3H) |
| | aromatic proton |

Example 8

Process for producing 4′-O-(1-cyclohexloxyethyl) adriamycin *a* and *b*

(a) 210 mg of p-toluenesulfonate of 14-O-acetyl adriamycin was dissolved in 4.2 ml of anhydrous dioxane ans 2.1 ml of anhydrous dimethyl sulfoxide, adding 0.84 ml of cyclohexyl vinyl ether and 0.417 ml of 0.1N p-toluenesulfonic acid-dioxane solution, and allowed to react while agitating for 20 min. at room temperature. Subsequently, the reaction mixture was followed by the same treatment as that mentioned in Example 5 (a) to obtain 56.9 mg of 14-O-acetyl-4′-O-cyclohexyloxyethyl adriamycin *a* and 60.7 mg of 14-O-acetyl-4′-O-cyclohexyloxyethyl adriamycin *b*.

(b) Deacetylation at the C—14 position

56.9 mg of 14-O-acetyl-4′-O-cyclohexyloxyethyl adriamycin *a* and 60.7 mg of 14-O-acetyl-4′-O-cyclohexloxyethyl adriamycin *b* respectively obtained in (a) were treated in the same manner as that mentioned in Example 5 (b) to obtain 20.7 mg of orange powder of 4′-O-cyclohexyloxyethyl adriamycin *a* and 24.2 mg or orange powder of 4′-O-cyclohexyloxyethyl adriamycin *b* respectively.

<u>Compound *a*</u>

| | |
|---|---|
| Melting point (°C): | 175—182 (decomposition) |
| Specific rotation (C=0.1 in chloroform) $[\alpha]_D^{25}$: | +178° |
| PMR ($CDCl_3$, ppm): | 1.32 (d, 3H) C5′—$CH_3$ |
| | 1.38 (d, 3H) C4′—$OCHCH_3$ |
| | 2.92 (d, 1H), 3.23 (d, 1H) } C10—$CH_2$ |
| | 4.05 (s, 3H) 4—$OCH_3$ |
| | 4.76 (s, 2H) C14—$CH_2$ |
| | 4.97 (q, 1H) C4′—$OCHCH_3$ |
| | 5.23 (bs, 1H) C7—H |
| | 5.49 (bs, 1H) C1′—H |
| | 7.30—8.00 (m, 3H) aromatic proton |

<u>Compound *B*</u>

| | |
|---|---|
| Melting point (°C): | 177—181 (decomposition) |
| Specific rotation (C=0.1 in chloroform) $[\alpha]_D^{25}$: | +172° |
| PMR ($CDCl_3$, ppm): | 1.26 (d, 3H) C5′—$CH_3$ |
| | 1.40 (d, 3H) C4′—$OCHCH_3$ |
| | 2.87 (d, 1H), 3.18 (d, 1H) } C10—$CH_2$ |
| | 4.02 (s, 3H) 4—$OCH_3$ |
| | 4.68 (q, 1H) C4′—$OCHCH_3$ |
| | 4.74 (s, 2H), C14—$CH_2$ |
| | 5.19 (bs, 1H) C7—H |
| | 5.49 (bs, 1H) C1′—H |
| | 7.26—7.94 (m, 3H) aromatic proton |

Example 9

Process for producing 4′-O-(tetrahydrofuranyl) daunomycin

56 mg (0.1 mmoles) of daunomycin hydrochloride was dissolved in dry dimethylformamide, adding 0.1 ml of dihydrofuran and a small amount of p-toluenesulfonic acid as a catalyst, and allowed to stand for 8 hours at room temperature (4′-O-tetrahydrofuranylation). The reaction mixture was

poured into 20 ml of water, adding sodium hydrogen carbonate to neutralize, and extracted with chloroform. The chloroform layer was dried over anhydrous sodium sulfate and concentrated to dryness to obtain red materials. Two bands of the product having Rf 0.29 and 0.31 were detected by preparative thin-layer chromatography using silica gel (Merck Co.) and a solvent system of chloroform: methanol (9:1), but these bands could not be completely separated from each other.

The bands corresponding to the product were scratched out from silica gel plate and eluted with a chloroform-methanol mixture (1:1), and the eluate was concentrated under reduced pressure to obtain 12.4 mg of 4'-O-(tetrahydrofuranyl) daunomycin as red powder.

Yield 21%

Melting point (°C): 201—204 (decomposition)

PMR ($CDCl_3$, ppm): 1.29(6'—H), 1.70—2.30(furan-3,4-H), 2.44(14—H), 4.11(4—H), 5.28(1'—H), 5.43(furan, anomeric), 5.54(7—H), 7.33—8.11(1—H — 3—H)

Example 10

Process for producing 4'-O-(6-acetoxymethyltetrahydropyranyl) daunomycin

112 mg (0.2 mmoles) of daunomycin hydrochloride was dissolved in 2.0 ml of dry dimethylformamide, adding 15 mg of p-toluenesulfonic acid and 0.3 ml of 2-acetoxy-methyl-3,4-dihydro-2H-pyran and allowed to stand over night at room temperature (4'-O-(6-acetoxymethyltetrahydropyranylation). The reaction mixture was poured into 20 ml of 1% sodium hydrogen carbonate aqueous solution and extracted with 30 ml of chloroform.

The aqueous layer was extracted twice with 10 ml of chloroform respectively. The combined chloroform layer was washed four times with 10 ml of water, dried over anhydrous sodium sulfate and concentrated under reduced pressure to obtain the crude product. The product was developed on a preparative thin-layer chromatogram (2 mm thickness, 20x20 cm, one plate) using a solvent system of chloroform-methanol (9:1), and the band corresponding to the product having Rf 0.47 was scratched out from silica gel thin-layer and re-extracted with a chloroform-methanol mixture (1:1). The resulted red extract was concentrated to dryness and 55.3 mg of 4'-O-(6-acetoxymethyltetrahydropyranyl) daunomycin was obtained as red powder.

Yield 40%

Melting point (°C): 198—201

PMR ($CDCl_3$, ppm): 1.21—1.42(6'—H), 2.07—2.11(OAc), 2.43(14—H), 4.10(4—OMe), 5.53(7—H), 7.29—8.06(1—H — 3H)

Example 11

Process for producing 4'-O-(6-methoxytetrahydropyranyl) daunomycin *a* and *b*

112 mg (0.2 mmoles) of daunomycin hydrochloride was dissolved in 3.0 ml of dry dimethylformamide, adding 0.15 ml of 2-methoxy-3,4-dihydro-2H-pyran and 10 mg of p-toluenesulfonic acid and allowed to stand overnight at room temperature (4'-O-(6-methoxytetrahydropyranylation). The reaction mixture was poured into 20 ml of water, adjusting the pH at 8.0 with sodium hydrogen carbonate and extracted four times with 10 ml of chloroform respectively. The chloroform extracts were combined, dried over anhydrous sodium sulfate and concentrated to dryness to obtain red residue. The residue was further purified by preparative thin-layer chromatography using silica gel (Merck Co. 2 mm thickness, 20x20 cm, one plate) and a solvent system of chloroform-methanol (9:1). A band of Rf 0.38 was scratched out from the silica gel plate, and re-extracted with a chloroform-methanol mixture (1:1). The resulting red extract was concentrated under reduced pressure to obtain 23.1 mg of compound *a* as red powder.

Yield 18%

Melting point (°C): 189—191

PMR ($CDCl_3$), ppm: 1.29(6'—H), 2.40(3—H), 3.45(5'—OMe), 4.09(4—OMe), 4.83(1'—H), 5.30(1'—H), 5.58(7—H), 7.28—8.08(1—H — 3—H)

19.4 mg of compound *b* was obtained as red powder according to the same method as that of compound *a*.

Yield 15%

Melting point (°C): 198—199

PMR ($CDCl_3$, ppm): 1.39(6'—H), 2.44(14—H), 3.46(pyran-OMe), 4.11(4—H), 4.89(pyran, anomeric), 5.22(1'—H), 5.53(7—H), 7.28—8.14(1—H — 3—H)

Example 12

Process for producing 4'-O-(6-carbomethoxytetrahydropyranyl) daunomycin

112 mg (0.2 mmoles) of daunomycin hydrochloride was dissolved in 3.0 ml of dry dimethylformamide, adding 0.1 ml of 2-carbomethoxy-3,4-dihydro-2H-pyran and 34 mg (0.2 mmoles) of p-toluenesulfonic acid, and allowed to stand for 10 hours at room temperature in the dark (4'-O-6-carbomethoxytetrahydropyranylation). The reaction mixture was poured into 20 ml of water, adjusting the pH at 8 with sodium hydrogen carbonate and extracted four times with 10 ml of chloroform respectively. The chloroform extracts were combined, washed twice with water and dried over anhydrous sodium sulfate. The chloroform extract was concentrated under reduced pressure, and the resulting residue was purified by preparative thin-layer chromatography using a silica gel plate (Merck Co.) and a solvent system of chloroform-methanol (9:1). New products were detected between 0.35 and 0.37 of Rf values on the TLC as a mixture of 4 components, and they could not be separated completely. The corresponding bands on the TLC were scratched out from the silica gel plate and extracted with a chloroform-methanol mixture (1:1). The extract was concentrated to dryness to obtain 25.9 mg of red powder.

Yield 19%

Melting point (°C): 190—193

PMR ($CDCl_3$, ppm): 1.23—1.40(6'—H), 2.43(14—H), 3.78($COOCH_3$), 4.09(4—OMe), 4.74, 5.07(pyran, anomeric), 5.31(1'—H), 5.54(7—H), 7.30—8.06(1—H — 3—H)

Example 13

Process for producing 4'-O-(1-ethyloxyethyl) daunomycin *a* and *b*

112 mg (0.2 mmoles) of daunomycin hydrochloride was dissolved in 3 ml of dry dimethylformamide, 0.1 ml of ethyl vinyl ether and 2 mg of p-toluenesulfonic acid and allowed to stand for 10 hours at room temperature (1-ethyloxyethylation). New spots of the product were observed at Rf 0.31 and 0.34 on the silica gel thin-layer chromatogram (solvent system, chloroform-methanol=9:1). The reaction mixture was poured into 20 ml of water, adjusting the pH at 8 with sodium hydrogen carbonate, and extracted with 30 ml of chloroform. The chloroform layer was dried and concentrated under reduced pressure. The resulting red residue was purified by preparative thin-layer chromatography using silica gel (Merck Co. thickness 2 mm, 20×20 cm, one plate) and a solvent system of chloroform-methanol (15:1). The band correspoonding to Rf 0.31 was scratched out, and extracted with a chloroform-methanol mixture (1:1). The red extract was concentrated to dryness to obtain 33.0 mg of red compound *a*.

Yield 27%

Melting point (°C): 208—210

PMR ($CDCl_3$ ppm): 1.76, 3.59(1'—OEt), 1.39(2'—H), 1.79(8—H), 2.41(14—H), 4.07(4—OMe), 4.64(1'—H), 5.25(1'—H), 5.51(7—H), 7.25—8.07(1—H — 3—H)

The band corresponding to Rf 0.34 was also treated according to the same method as that mentioned above, and 31.8 mg of compound *b* was obtained as red powder.

Yield 26%

Melting point (°C): 200—204

PMR ($CDCl_3$, ppm): 1.20, 3.64(1'—OEt), 1.40(2'—H), 2.41(14—H), 4.09(4—H), 4.94(1'—H), 5.26(1'—H), 5.50(7—H), 7.26—8.07(1—H — 3—H)

Example 14

Process for producing 4'-O-(1-isobutyloxyethyl) daunomycin *a* and *b*

60 mg (0.106 mmoles) of daunomycin hydrochloride was dissolved in 12 ml of absolute THP and 1.5 ml of absolute DMSO, adding 1.2 ml of 2-isobutyl vinyl ether and 0.21 ml of 0.1N p-toluenesulfonic

acid-THF solution, and agitated for three hours at room temperature. The reaction mixture was dissolved in 100 ml of ethylacetate, washed with 100 ml of 5% sodium chloride aqueous solution containing 0.1N sodium hydrogen carbonate, and further washed with 5% sodium chloride aqueous solution (100 ml ×2). The solvent layer was dried over anhydrous sodium sulfate and concentrated under reduced pressure. The resulting oily substance was treated in the same manner as that mentioned in Example 13 to obtain 16.0 mg of 4'-O-(1-isobutyloxyethyl) daunomycin *a* and 19.1 mg of 4'-O-(1-isobutyloxyethyl) daunomycin *b*.

Compound *a*

| | |
|---|---|
| Melting point (°C): | 143—148 |
| Specific rotation (C=0.1 in chloroform) $[\alpha]_D^{25}$: | +190° |
| PMR ($CDCl_3$, ppm): | 0.92 (d, 6H) $CH(CH_3)_2$ |
| | 1.33 (d, 3H) C5'—$CH_3$ |
| | 1.37 (d, 3H) C4'—$OCHCH_3$ |
| | 2.41 (s, 3H) C13—$CH_3$ |
| | 2.90 (d, 1H), 3.22 (d, 1H) } C10—$CH_2$ |
| | 3.27 (t, 2H) $OCH_3CH$ |
| | 3.62 (bs, 1H) C4'—H |
| | 4.07 (s, 3H) C4—$OCH_3$ |
| | 4.89 (q, 1H) C4'—$OCHCH_3$ |
| | 5.28 (bs, 1H) C7—H |
| | 5.52 (bs, 1H) C1'—H |
| | 7.31—8.06 (m, 3H) aromatic proton |

Compound *b*

| | |
|---|---|
| Melting point (°C): | 141—146 |
| Specific rotation (C=0.1 in chloroform) $[\alpha]_D^{25}$: | +185° |
| PMR ($CDCl_3$, ppm): | 0.90 (d, 6H) $CH(CH_3)_2$ |
| | 1.25 (d, 3H) C5'—$CH_3$ |
| | 1.39 (d, 3H) C4'—$OCHCH_3$ |
| | 2.40 (s, 3H) C13—$CH_3$ |
| | 2.89 (d, 1H), 3.22 (d, 1H) } C10—$CH_2$ |
| | 3.26 (t, 2H) $OCH_2CH$ |

3.54 (bs, 1H) C4′—H

4.06 (s, 3H) C4—$OCH_3$

4.61 (q, 1H) C4′—OC*H*$CH_3$

5.27 (bs, 1H) C7—H

5.52 (bs, 1H) C1′—H

7.31—8.04 (m, 3H)

aromatic proton

Example 15

Process for producing 4′-O-(1-butyloxyethyl) daunomycin *a* and *b*

100 mg (0.177 mmoles) of daunomycin hydrochloride was dissolved in a mixture of 10 ml of absolute THF and 2 ml of absolute DMSO, adding 3 ml of n-butyl vinyl ether and 0.88 ml (0.088 mmoles) of 0.1N p-toluenesulfonic acid-THF solution, and agitated for two hours at room temperature. According to the method of Example 14, 27.1 mg of 4′-O-(1-butyloxyethyl) daunomycin *a* and 34.8 mg of 4′-O-(1-butyloxyethyl) daunomycin *b* were separated.

Compound *a*

Melting point (°C): 141—145

Specific rotation (C=0.1 in chloroform) $[\alpha]_D^{25}$: +167°

PMR ($CDCl_3$, ppm):

0.91 (t, 3H) —$CH_2C\textit{H}_3$

1.32 (d, 3H) C5′—$CH_3$

1.37 (d, 3H) C4′—OCHC*H*$_3$

2.41 (s, 3H) C13—$CH_3$

2.92 (d, 1H) } C10—$CH_2$
3.24 (d, 1H) }

3.54 (m, 3H) C4′—H, $OCH_2$—

4.07 (s, 3H) C4—$OCH_3$

4.91 (q, 1H) C4′—OC*H*$CH_3$

5.27 (bs, 1H) C7—H

5.52 (bs, 1H) C1′—H

7.31—8.06 (m, 3H)

aromatic proton

Compound *b*

Melting point (°C): 138—142

Specific rotation (C=0.1 in chloroform) $[\alpha]_D^{25}$: +207°

PMR ($CDCl_3$, ppm):

0.89 (t, 3H) —$CH_2CH_3$

1.27 (d, 3H) C5'—$CH_3$

1.39 (d, 3H) C4'—$OCHCH_3$

2.40 (s, 3H) C13—$CH_3$

2.86 (d, 1H) } C10—$CH_2$
3.20 (d, 1H) }

3.52 (m, 3H) C4'—H, $OCH_2$—

4.06 (s, 3H) C4—$OCH_3$

4.61 (q, 1H) C4'—$OCHCH_3$

5.25 (bs, 1H) C7—H

5.51 (bs, 1H) C1'—H

7.30—8.01 (m, 3H)

aromatic proton

**Claims**

1. An anthracycline derivative of the general formula I:

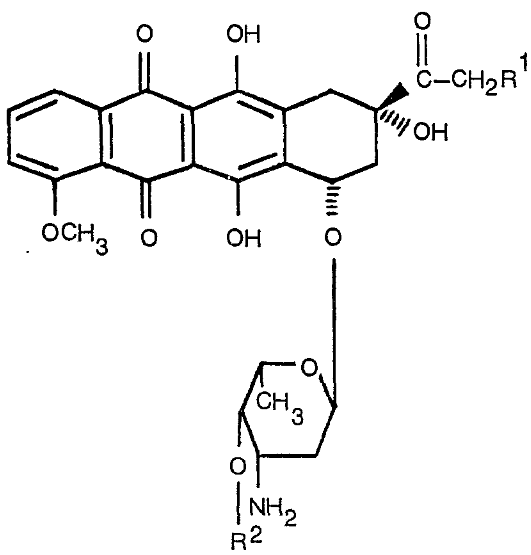

(I)

wherein $R^1$ is a hydrogen atom or a hydroxyl group, and $R^2$ is an 1-alkyloxyethyl, a tetrahydrofuran-2-yl, a 6-methoxytetrahydropyran-2-yl, a 6-carbomethoxytetrahydropyran-2-yl or a 6-acetoxymethyltetrahydropyran-2-yl group or an acid addition salt thereof.

2. A process for producing a 14-O-deacylated derivative of the general formula III:

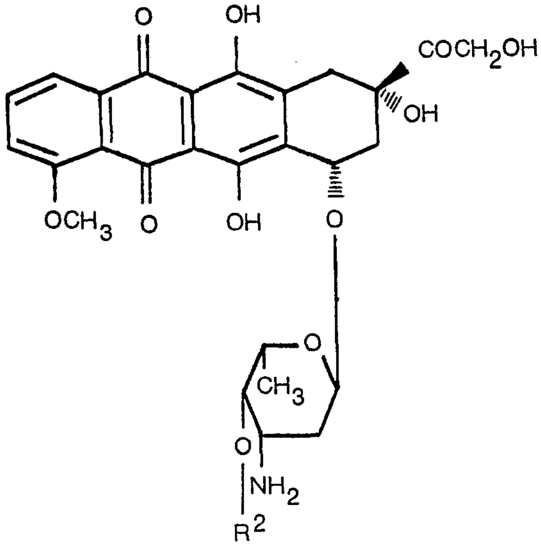

(III)

wherein $R^2$ is an 1-alkyloxyethyl, a tetrahydrofuran-2-yl, a 6-methoxytetrahydropyran-2-yl, a 6-carbomethoxytetrahydropyran-2-yl or a 6-acetoxymethyltetrahydropyran-2-yl group or an acid addition salt thereof which comprises introducing an alkyloxyethyl, a tetrahydrofuranyl, a 6-methoxytetrahydropyranyl, a 6-carbomethoxytetrahydropyranyl or a 6-acetoxymethyltetrahydropyranyl group into the hydroxyl group at C—4′ position of an anthracycline derivative of the formula II-a:

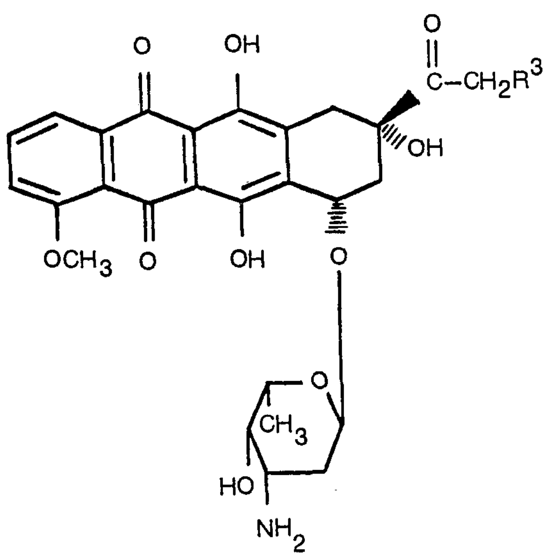

(II-a)

wherein $R_3$ is an alkanoyloxy group having 2 to 7 carbon atoms or a phenylacetyloxy group or an acid addition salt thereof by reaction with dihydrofuran, an appropriate dihydropyran derivative or an alkyl vinyl ether which contains said corresponding group to be introduced and is capable of reacting with a hydroxyl group, in an inert organic solvent and in the presence of an acid catalyst to form said anthracycline derivative of the general formula II:

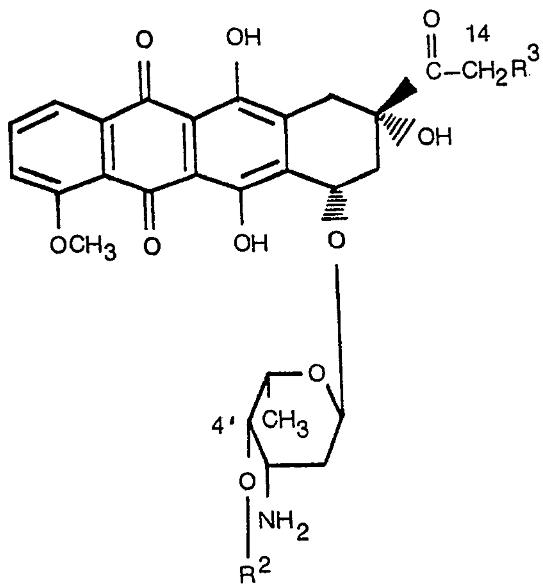

(II)

wherein $R^3$ is an alkanoyloxy group having 2 to 7 carbon atoms or a phenylacetyloxy group and $R^2$ has the same definition as mentioned above or an acid addition salt thereof and further comprises eliminating the alkanoyl group or the phenylacetyl group at the C—14 position of said derivative of the general formula II or an acid addition salt thereof by hydrolytic deacylation to form said anthracycline derivative of the general formula III.

3. A process for producing an anthracycline derivative of the general formula V:

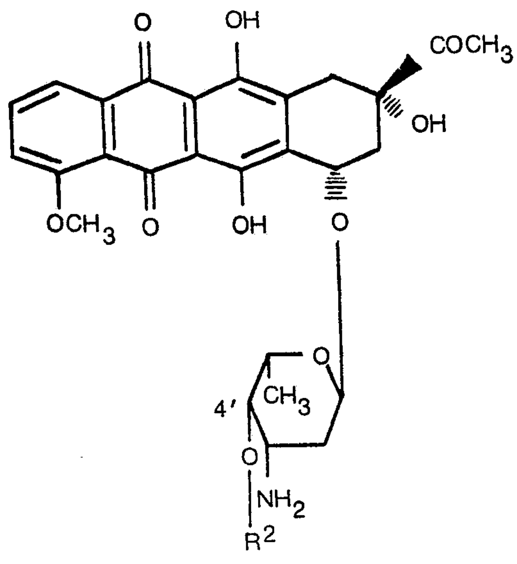

(V)

wherein $R^2$ is a 1-alkyloxyethyl, a tetrahydrofuran-2-yl, a 6-methoxytetrahydropyran-2-yl, a 6-carbomethoxytetrahydropyran-2-yl or a 6-acetoxymethyltetrahydropyran-2-yl group or an acid addition salt thereof which comprises introducing an alkyloxyethyl, a tetrahydrofuranyl, a 6-methoxytetrahydropyranyl, a 6-carbomethoxytetrahydropyranyl or a 6-acetoxymethyltetrahydropyranyl group into the hydroxyl group at the C—4′ position of the anthracycline glycoside of the general formula IV:

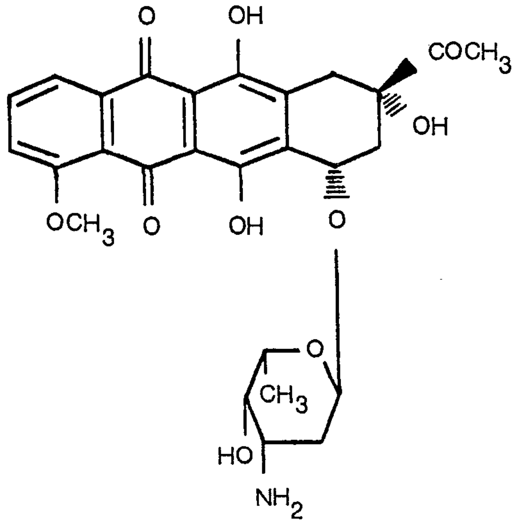

(IV)

or an acid addition salt thereof by reaction with dihydrofuran, an appropriate dihydropyran derivative or an alkyl vinyl ether which contains said corresponding group to be introduced and is capable of reaction with a hydroxyl group, in an inert organic solvent and in the presence of an acid catalyst to form said anthracycline derivative.

4. A pharmaceutical composition comprising a therapeutically effective amount of a compound as claimed in claim 1 and a pharmaceutical carrier or diluent.

**Patentansprüche**

1. Anthracyclinderivat der allgemeinen Formel I:

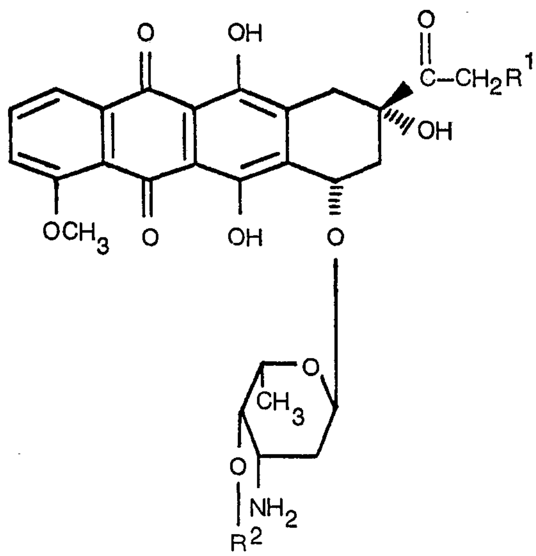

(I)

worin $R^1$ ein Wasserstoffatom oder eine Hydroxylgruppe ist und $R^2$ eine 1-Alkyloxyethyl-, Tetrahydrofuran-2-yl-, 6-Methoxytetrahydropyran-2-yl-, 6-Carbomethoxytetrahydropyran-2-yl oder 6-Acetoxymethyltetrahydropyran-2-yl- gruppe bedeutet, oder ein Säureadditionssalz davon.

2. Verfahren zur Herstellung eines 14-O-entacylierten Derivats der allgemeinen Formel III:

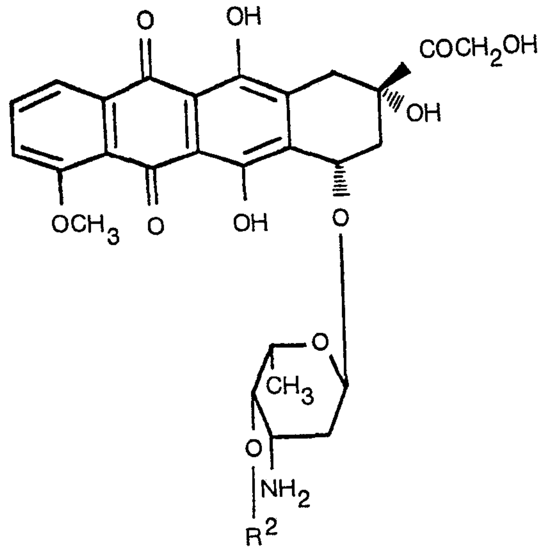

(III)

worin $R^2$ eine 1-Alkyloxyethyl-, Tetrahydrofuran-2-yl-, 6-Methoxytetrahydropyran-2-yl-, 6-Carbomethoxytetrahydropyran-2-yl- oder 6-Acetoxymethyltetrahydropyran-2-yl-gruppe bedeutet oder eines Säureadditionssalzes davon, dadurch gekennzeichnet, daß eine Alkyloxyethyl-, Tetrahydrofuranyl-, 6-Methoxytetrahydropyranyl-, 6-Carbomethoxytetrahydropyranyl- oder 6-Acetoxymethyltetrahydropyranylgruppe in die Hydroxylgruppe in der C—4'-Stellung eines Anthracyclinderivats der Formel II-a;

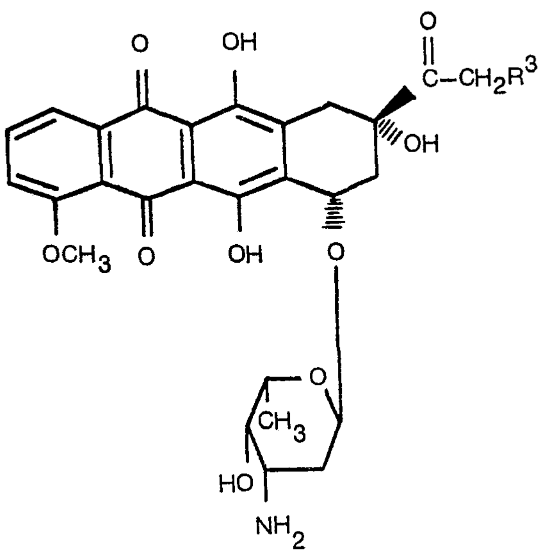

(II-a)

worin $R^3$ eine Alkanoyloxygruppe mit 2 bis 7 Kohlenstoffatomen oder eine Phenylacetyloxygruppe ist, oder eines Säureadditionssalzes davon durch Umsetzen mit Dihydrofuran, einem entsprechenden Dihydropyranderivat oder einem Alkylvinylether, der die entsprechende einzuführende Gruppe enthält und mit einer Hydroxylgruppe zu reagieren vermag, in einem inerten organischen Lösungsmittel sowie in Gegenwart eines sauren Katalysators unter Bildung eines Anthracyclinderivats der allgemeinen Formel II:

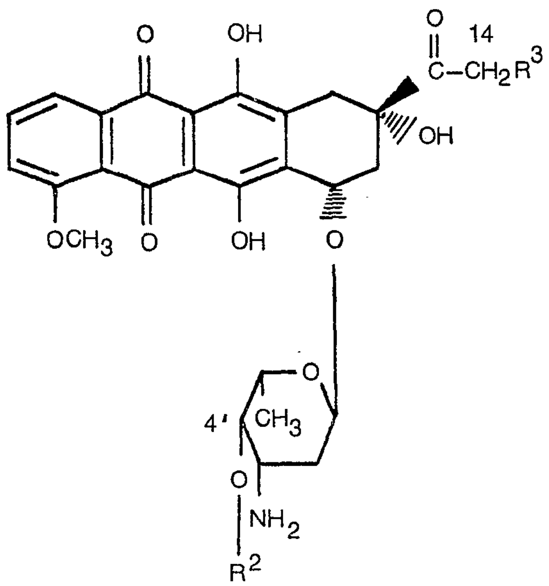

(II)

worin $R^3$ eine Alkanoyloxygruppe mit 2 bis 7 Kohlenstoffatomen oder eine Phenylacetyloxygruppe ist und $R^2$ die gleiche vorstehend angegebene Definition besitzt, oder eines Säureadditionssalzes davon eingeführt wird und außerdem die Alkanoylgruppe der Phenylacetylgruppe in der C—14-Position des Derivats der allgemeinen Formel II oder eines Säureadditionssalzes davon durch hydrolytische Entacylierung unter Bildung eines Anthracyclinderivats der allgemeinen Formel III eliminiert wird.

3. Verfahren zur Herstellung eines Anthracyclinderivats der allgemeinen Formel V:

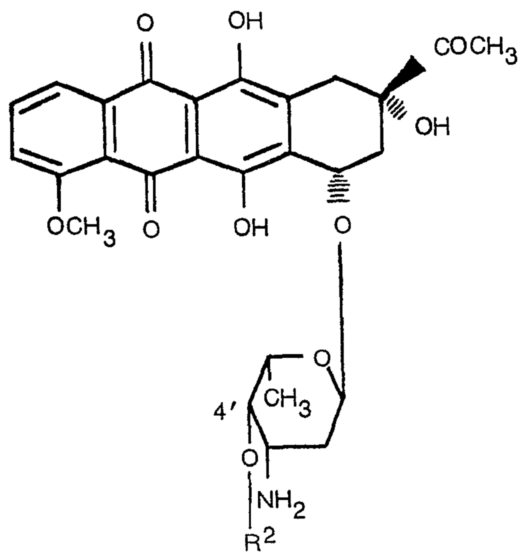

(V)

worin $R^2$ eine 1-Alkyloxyethyl-, Tetrahydrofuran-2-yl, 6-Methoxytetrahydropyran-2-yl-, 6-Carbomethoxytetrahydropyran-2-yl- oder 6-Acetoxymethyltetrahydropyran-2-yl-gruppe ist oder eines Säureadditionssalzes davon, dadurch gekennzeichnet, daß eine Alkyloxyethyl-, Tetrahydrofuranyl-, 6-Methoxytetrahydropyranyl-, 6-Carbomethoxytetrahydropyranyl- oder 6-Acetoxymethyltetrahydropyranylgruppe in die Hydroxylgruppe in der C—4'-Position eines Anthracyclinglykosids der allgemeinen Formel IV:

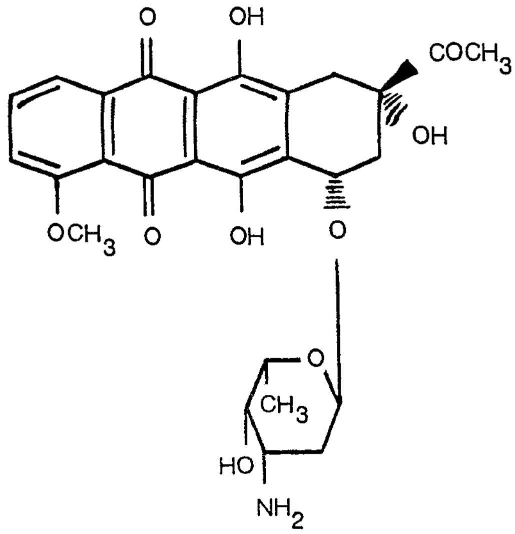

(IV)

oder eines Säureadditionssalzes davon durch Umsetzung mit Dihydrofuran, einem entsprechenden Dihydropyranderivat oder einem Alkylvinylether, der die entsprechend einzuführende Gruppe enthält und zu einer Reaktion mit der Hydroxylgruppe befähigt ist, in einem inerten organischen Lösungsmittel sowie in Gegenwart eines sauren Katalysators unter Bildung des Anthracyclinderivats eingeführt wird.

4. Pharmazeutisches Mittel, gekennzeichnet durch eine therapeutisch wirksame Menge einer Verbindung gemäß Anspruch 1 sowie einen pharmazeutischen Träger oder ein pharmazeutisches Verdünnungsmittel.

## Revendications

1. Un dérivé d'anthracycline de formule générale I:

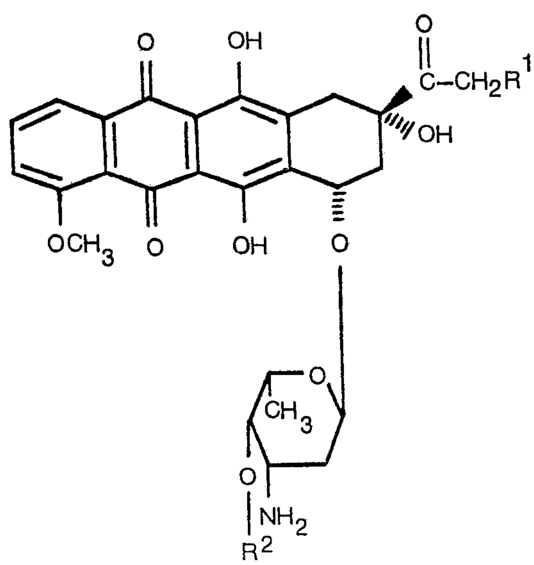

(I)

où $R^1$ est un atome d'hydrogène ou un groupe hydroxy, et $R^2$ est un groupe 1-alcoxyéthyle, tétrahydrofuranne-2-yle, 6-méthoxytétrahydropyranne-2-yle, 6-méthoxycarbonyltétrahydropyranne-2-yle ou 6-acétoxyméthyltétrahydropyranne-2-yle, ou un sel d'addition d'acide correspondant.

2. Un procédé pour produire un dérivé 14-O-désacylé de formule générale III:

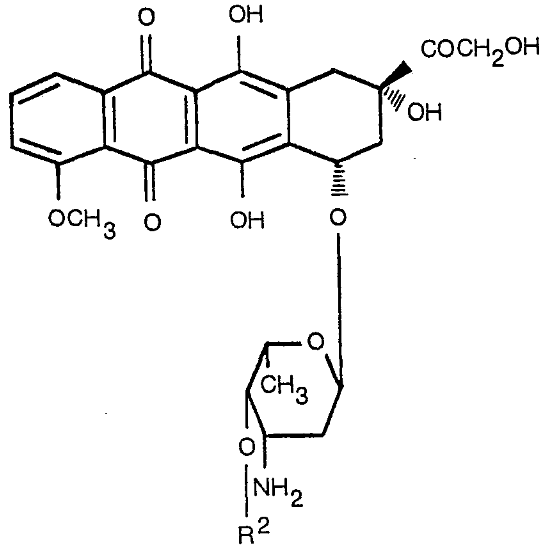

(III)

où $R^2$ est un groupe 1-alcoxyéthyle, tétrahydrofuranne-2-yle, 6-méthoxytétrahydropyranne-2-yle, 6-méthoxycarbonyltétrahydropyranne-2-yle ou 6-acétoxyméthyltétrahydropyranne-2-yle, ou un sel d'addition d'acide correspondant, qui comprend l'introduction d'un groupe alcoxyéthyle, tétrahydrofuryle, 6-méthoxytétrahydropyrannyle, 6-méthoxycarbonyltétrahydropyrannyle ou 6-acétoxyméthyltétrahydropyrannyle, dans le groupe hydroxy en position C—4' d'un dérivé d'anthracycline de formule II-a:

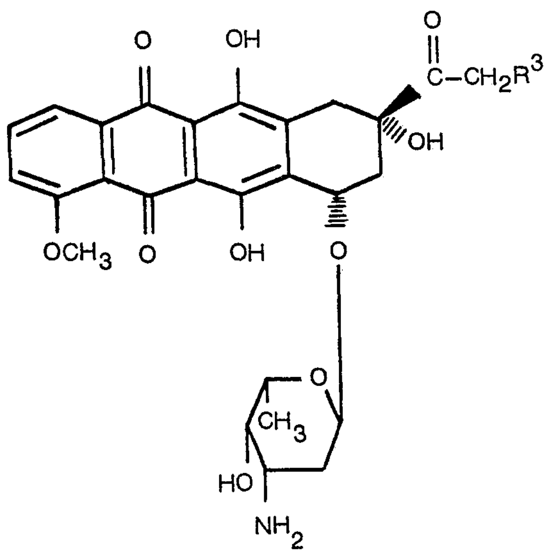

(II-a)

où $R^3$ est un groupe alcanoyloxy ayant 2 à 7 atomes de carbone ou un groupe phénylacétoxy, ou un sel d'addition d'acide correspondant, par réaction avec le dihydrofuranne, un dérivé approprié du dihydropyranne ou un éther alkylvinylique qui contient ledit groupe correspondant à introduire et qui est capable de réagir avec un groupe hydroxy, dans un solvant organique inerte et en présence d'un catalyseur acide pour former ledit dérivé d'anthracycline de formule générale II:

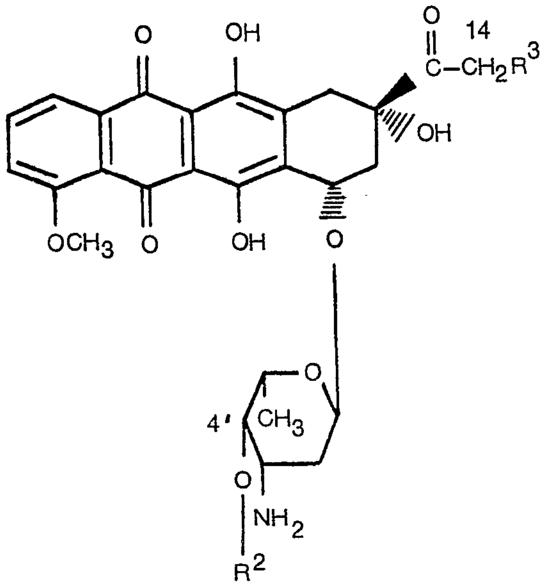

(II)

où $R^3$ est un groupe alcanoyloxy ayant 2 à 7 atomes de carbone ou un groupe phénylacétoxy et $R^2$ a la même définition que ci-dessus, ou un sel d'addition d'acide correspondant, et qui comprend de plus l'élimination du groupe alcanoyle ou du groupe phénylacétyle en position C—14 dudit dérivé de formule générale II ou d'un sel d'addition d'acide correspondant par désacylation hydrolytique pour former ledit dérivé d'anthracycline de formule générale III.

3. Un procédé pour produire un dérivé d'anthracycline de formule générale V:

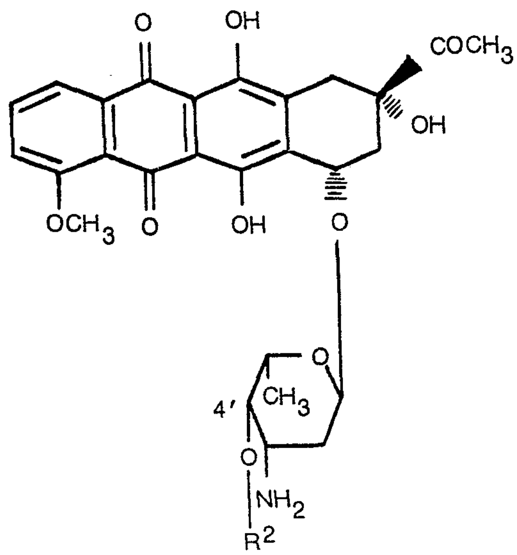

(V)

où $R^2$ est un groupe 1-alcoxyéthyle, tétrahydrofuranne-2-yle, 6-méthoxytétrahydropyranne-2-yle, 6-méthoxycarbonyltétrahydropyranne-2-yle ou 6-acétoxyméthyltétrahydropyranne-2-yle, ou un sel d'addition d'acide correspondant, qui comprend l'introduction d'un groupe alcoxyéthyle, tétrahydrofuryle, 6-méthoxytétrahydropyrannyle, 6-méthoxycarbonyltétrahydropyrannyle ou 6-acétoxyméthyltétrahydropyrannyle dans le groupe hydroxy en position C—4' de l'anthracyclineglucoside de formule générale IV:

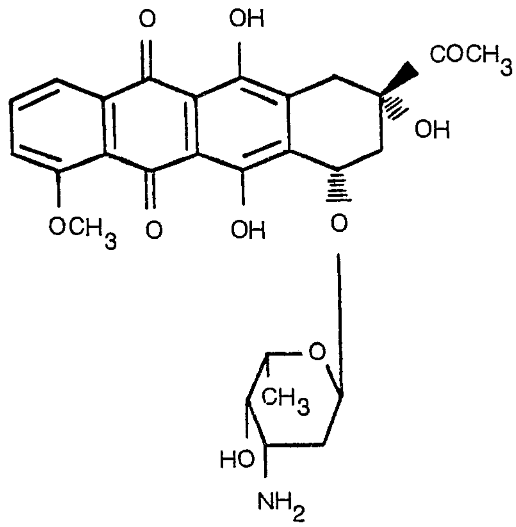

(IV)

ou un sel d'addition d'acide correspondant par réaction avec le dihydrofuranne, un dérivé approprié de dihydropyranne ou un éther alkylvinylique qui contient ledit groupe correspondant à introduire et qui est capable de réagir avec un groupe hydroxy, dans un solvant organique inerte et en présence d'un catalyseur acide pour former ledit dérivé d'anthracycline.

4. Une composition pharmaceutique comprenant une quantité thérapeutique efficace d'un composé comme revendiqué dans la revendication 1 et un véhicule ou diluant pharmaceutique.